# FASCICULE DE BREVET EUROPEEN

(11) **EP 2 449 370 B1**
(45) Date de publication et mention de la délivrance du brevet: **11.03.2020**
(21) Numéro de dépôt: 10734185.1
(22) Date de dépôt: 30.06.2010
(51) Int. Cl.: G01N 27/447

(54) **ANALYSE ET DOSAGE D'HÉMOGLOBINES GLYQUÉES PAR ÉLECTROPHORÈSE CAPILLAIRE, COMPOSITIONS TAMPON ET KITS POUR ÉLECTROPHORÈSE CAPILLAIRE**
ANALYSE UND TESTEN GLYKIERTER HÄMOGLOBINE MITTELS KAPILLARELEKTROPHORESE, PUFFERZUSAMMENSETZUNG UND KITS FÜR KAPILLARELEKTROPHORESE
ANALYSIS AND ASSAY OF GLYCATED HEMOGLOBINS BY CAPILLARY ELECTROPHORESIS, BUFFER COMPOSITIONS, AND KITS FOR CAPILLARY ELECTROPHORESIS

(30) Priorité: 01.07.2009 FR 0903220
(43) Date de publication de la demande: 09.05.2012
(73) Titulaire: SEBIA, 91090 Lisses (FR)
(72) Inventeur: DESCHAMPS, Gérald, 77760 Ury (FR); ROBERT, Frédéric, 91540 Mennecy (FR); SIMONIN, Denis, 91000 Evry (FR)
(74) Mandataire: Desaix, Anne
(86) Numéro de dépôt international: PCT/FR2010/000481
(87) Numéro de publication internationale: WO 2011/001045

(56) Documents cités:
- EP-A1- 1 659 398
- WO-A1-96/22524
- WO-A1-98/40750
- SONG S Y ET AL: "Boronic acid-modified thin film interface for specific binding of glycated hemoglobin (HbA1c) and electrochemical biosensing" SENSORS AND ACTUATORS B, ELSEVIER SEQUOIA S.A., LAUSANNE, CH, vol. 140, no. 1, 18 juin 2009 (2009-06-18) , pages 233-239, XP026148990 ISSN: 0925-4005 [extrait le 2009-05-07]

## Description

La présente demande concerne le domaine de l'analyse et du dosage d'hémoglobines glyquées, en particulier de l'hémoglobine A_{1c}, par électrophorèse capillaire.

Sont décrites une méthode d'analyse par électrophorèse capillaire d'un échantillon biologique comprenant une ou plusieurs hémoglobine(s) et en particulier une ou plusieurs hémoglobine(s) glyquée(s), ainsi que des compositions tampon appropriées pour cette analyse, et des trousses d'analyse et de dosage d'hémoglobines glyquées par électrophorèse capillaire. L'invention porte plus particulièrement sur une méthode d'analyse par électrophorèse capillaire d'une ou plusieurs hémoglobine(s) glyquée(s) comportant au moins une chaîne de globine bêta comportant un résidu glucose lié à l'acide aminé se trouvant en position N-terminale, telle que définie dans l'une des revendications 1 à 11, sur une composition tampon telle que définie en revendication 12 ou 13, sur une trousse telle que définie en revendication 14, et sur l'utilisation d'une composition tampon ou d'une trousse pour la séparation, par électrophorèse capillaire, d'une ou plusieurs hémoglobine(s) glyquée(s) comportant au moins une chaîne de globine bêta comportant un résidu glucose lié à l'acide aminé se trouvant en position N-terminale, d'autres protéines, telle que définie en revendication 15, notamment, selon la revendication 16, pour le diagnostique du diabète chez un humain ou un mammifère non humain et/ou pour le suivi de l'équilibre glycémique d'un mammifère humain ou non humain.

L'hémoglobine (Hb) est une molécule globulaire comprenant généralement quatre sous-unités constituées chacune d'une chaîne polypeptidique conjuguée à une portion hème. Les chaînes polypeptidiques sont désignées collectivement sous le nom de portion globine de la molécule d'hémoglobine.

Toutes les hémoglobines humaines sont constituées de quatre chaînes polypeptidiques deux à deux identiques. Chez un humain adulte, trois types d'hémoglobine sont normalement présents : l'hémoglobine A (HbA), très largement majoritaire (elle représente généralement environ 97% de l'hémoglobine chez un adulte), constituée de 2 chaînes alpha et de deux chaînes bêta (hémoglobine α₂β₂), l'hémoglobine A2 (HbA2 ; elle représente généralement environ 1-3% de l'hémoglobine chez un adulte) constituée de deux chaînes alpha et de deux chaînes delta (hémoglobine α₂δ₂), et l'hémoglobine fœtale (HbF), constituée de deux chaînes alpha et de deux chaînes gamma (hémoglobine α₂γ₂), qui ne subsiste plus qu'à l'état de traces (généralement inférieures à 1% de l'hémoglobine chez un adulte) et reste limitée à une population cellulaire restreinte (les "cellules F").

L'hémoglobine contient des espèces dites mineures, qui sont des hémoglobines glyquées.

L'hémoglobine glyquée (également appelée hémoglobine glycosylée ou encore glycohémoglobine) correspond à l'ensemble des molécules d'hémoglobine modifiées par fixation d'oses, principalement de glucose, sur les fonctions aminées (NH₂) de la globine ; le groupement NH₂ d'une hémoglobine se condense avec un groupement aldéhyde issu d'un sucre réducteur. Cette réaction, qui est non enzymatique, est ensuite stabilisée par un réarrangement d'Amadori. Les sites de glycation potentiels sont les acides aminés N-terminaux des quatre chaînes de globine de l'hémoglobine (chaînes α et chaînes β, δ, ou γ selon le type d'hémoglobine) et les groupes amino-epsilon libres (en particulier ceux des résidus de lysine) au sein des chaînes de globine de l'hémoglobine.

Il existe de nombreuses formes d'hémoglobine glyquée en fonction du nombre d'oses fixés, de la nature des chaînes de globine et des oses fixés sur ces chaînes et de l'emplacement de ces oses sur les chaînes de globine.

Le terme d'hémoglobine glyquée totale est utilisé lorsque l'on considère les molécules d'hémoglobine glyquées sur un quelconque résidu NH2.

Le terme d'hémoglobine A1 (HbA1) est réservé aux molécules d'hémoglobine A ayant une molécule d'ose liée à l'acide aminé N-terminal d'une ou des deux chaînes bêta de la protéine. La fraction A1, hétérogène, comprend notamment les fractions mineures A₁ₐ, A_{1b} et surtout A_{1c}. L'hémoglobine A₁ₐ (HbA₁ₐ) regroupe l'hémoglobine A₁ₐ₁ (HbA₁ₐ₁), sur laquelle l'ose lié à l'acide aminé N-terminal est du fructose 1-6 diphosphate, et l'hémoglobine A₁ₐ₂ (HbA₁ₐ₂), sur laquelle l'ose lié à l'acide aminé N-terminal est du glucose-6-phosphate. Dans le cas de l'hémoglobine A_{1b} (HbA_{1b}), l'ose lié à l'acide aminé N-terminal est du pyruvate. Enfin, l'ose lié à l'acide aminé N-terminal des chaînes bêta de l'hémoglobine A_{1c} (HbA_{1c}) est du glucose ; l'HbA_{1c} est obtenue par condensation d'une molécule de glucose avec le groupement NH₂ de la valine N-terminale de la chaîne β, pour former une céto-amine stable, issue d'un réarrangement (réarrangement d'Amadori) d'une aldimine instable préalablement formée (dénommée HbA_{1c} labile ou preA_{1c}).

Enfin, on regroupe généralement sous le terme d'hémoglobine A₀ (HbAo) les hémoglobines A non glyquées et les hémoglobines A glyquées ne comportant pas d'ose lié sur l'acide aminé en position N-terminale des chaînes β.

La glycation des protéines (dont l'hémoglobine glyquée et en particulier l'hémoglobine A_{1c} parmi tant d'autres) est provoquée par la trop forte concentration de sucres dans le sang (comme c'est le cas dans le diabète). La glycation des protéines altère leurs fonctions, ce qui provoque des lésions cellulaires, tissulaires, et un vieillissement vasculaire et participe au développement de plusieurs maladies, telles que l'artériosclérose, l'insuffisance rénale, la rétinopathie diabétique et la cataracte. Une fois glycosylée, l'hémoglobine transporte moins bien l'oxygène.

Parmi tous les groupes et sous-groupes d'hémoglobine, l'HbA_{1c} présente un intérêt particulier parce qu'elle sert de marqueur à long terme de l'état diabétique des patients. La formation de l'HbA_{1c} est dépendante de la glycémie (concentration de glucose dans le sang). Elle intervient pendant toute la durée de vie des globules rouges, qui est normalement de 120 jours. La concentration sanguine d'HbA_{1c} traduit donc une moyenne de la glycémie sur les 2-3 mois qui précèdent le dosage. L'élévation de la concentration sanguine d'HbA_{1c} est révélatrice d'une hyperglycémie prolongée au cours de cette période. En effet, la concentration sanguine d'HbA_{1c} n'est pas influencée par de brèves fluctuations dans les taux de glucose sanguin. Ainsi, en mesurant le taux d'HbA_{1c} entre deux dates, on peut suivre l'évolution d'un diabète.

Chez le patient diabétique, le but est d'obtenir un taux d'HbA_{1c} aussi proche que possible des chiffres caractérisant un bon équilibre de la glycémie. La valeur de référence chez un humain non diabétique ayant une glycémie normale est de 4 à 6 % d'HbA_{1c} dans le sang par rapport à la concentration totale d'hémoglobines A (hémoglobines glyquées et hémoglobines non glyquées) (soit 20 à 42 mmol/moles ; Panteghini et al., 2007).

La détection des hémoglobines glyquées, en particulier la détection d'une augmentation du taux d'HbA_{1c}, présente donc un intérêt manifeste pour le diagnostique du diabète (de type 1 ou de type 2) ou le suivi de patients diabétiques et en particulier pour évaluer l'efficacité d'un traitement contre le diabète (de type 1 ou de type 2).

Pourtant, l'interprétation des résultats est parfois délicate, car de nombreux facteurs peuvent fausser les résultats, en particulier la présence d'hémoglobines anormales.

Des hémoglobines dites « anormales » ou variants, désignées notamment par les lettres S, C, D, E, H et J, sont en effet rencontrées chez certains patients. L'hémoglobine A est alors partiellement ou totalement remplacée par une ou plusieurs hémoglobines anormales. Elles proviennent notamment d'une synthèse réduite de certaines chaînes de globine et/ou d'une modification de structure des chaînes α, β, γ ou δ, par substitution d'un acide aminé par un autre. Ainsi, une modification intervenant au niveau de la chaîne β peut donner naissance, par exemple, à des hémoglobines S ou C (hémoglobines α₂β₂ dans lesquelles l'acide glutamique en position 6 dans la chaîne β est substitué respectivement par une valine ou une lysine).

Ces hémoglobines anormales sont également susceptibles d'être glyquées. Ainsi, il existe notamment les hémoglobines S_{1c} (HbS_{1c}) et C_{1c} (HbC_{1c}) qui, comme l'HbA_{1c}, comportent une molécule de glucose fixée sur la valine N-terminale sur des chaînes β (Abraham et al., 1984).

Les méthodes d'analyse et de dosage de l'hémoglobine glyquée et en particulier de l'hémoglobine HbA_{1c}, peuvent être classées en 2 grandes catégories.

La première catégorie, majoritaire, inclut des méthodes telles que les immuno-essais ou les chromatographies d'affinité. Elle est basée sur les caractéristiques structurelles de la molécule de sucre liée à l'hémoglobine. A titre d'exemple, la publication Wilson et al., 1993 et le brevet US 6,162,645 décrivent une méthode de dosage par chromatographie d'affinité de l'hémoglobine glyquée se trouvant dans un échantillon de sang humain. Ladite méthode repose sur l'utilisation d'une phase solide positivement chargée couplée, par l'intermédiaire d'un composé polyanionique (par exemple de l'acide polyacrylique), à de l'acide boronique, de l'acide phénylboronique, de l'acide borique ou un composé boronate similaire. Lorsqu'un échantillon de sang à analyser est incubé avec cette phase solide, les résidus glucose des molécules d'hémoglobine glyquée présentes dans cet échantillon se complexent avec le composé boronate. Les molécules d'hémoglobine glyquée se trouvent ainsi greffées sur la phase solide. On peut alors quantifier la proportion d'hémoglobine glyquée immobilisée sur la phase solide par rapport à la proportion d'hémoglobine non immobilisée. Ce dosage est réalisé soit en mesurant l'extinction de la fluorescence (Wilson et al., 1993), soit en utilisant des anticorps marqués dirigés contre l'hémoglobine humaine (US 6,162,645). Song S Y et al. dans « Boronic acid-modified thin film interface for specific binding of glycated hemoglobin (HbA1c) and electrochemical biosensing » Sensors and Actuators B, Elsevier Sequoia S.A., vol. 140, no. 1, 2009, pages 233-239 décrit également une méthode de détection de la fraction HbA_{1c}, précisément, des hémoglobines, employant comme agent de capture des électrodes sur lesquelles des acides boroniques sont greffés via des dendrimères. Ce document discute plus particulièrement la manière selon laquelle fonctionnaliser l'électrode, afin de permettre le biosensing recherché.

La deuxième catégorie de méthodes d'analyse et de dosage de l'hémoglobine glyquée inclut la chromatographie d'échange ionique ou l'électrophorèse. Elle exploite les caractéristiques physico-chimiques des molécules et repose sur la différence de charge existant entre les protéines glyquées et les protéines non glyquées.
Parmi les différentes méthodes analytiques permettant un dosage de l'hémoglobine glyquée décrites dans la littérature, les méthodes électrophorétiques se divisent en plusieurs catégories : les analyses sur gel incluent l'analyse en focalisation isoélectrique sur gel de polyacrylamide (Beccaria, 1978 ; Simon, 1982; Stickland, 1982 ; Bosisio, 1994) et l'analyse sur gel d'agarose (US 5,246,558 ; US 4,222,836 ; Menard, 1980). Les analyses en électrophorèse capillaire incluent l'analyse en focalisation isoélectrique (Molteni, 1994 ; Hempe, 1994), l'analyse en solution libre faisant intervenir un anticorps anti-A_{1c} (US 5,431,793), l'analyse en solution libre en capillaire nu (US 5,599,433 et WO 96/22524) et l'analyse en solution libre avec revêtement (« coating ») dynamique (EP 0 733 900 A2 ; US 5,611,903). On connaît aussi de EP 1 659 398 une méthode d'électrophorèse capillaire employant un tampon d'analyse zwitterionique associé à un ralentisseur de flux pour obtenir une séparation par électrophorèse capillaire entre les formes HbA2, HbC, HbD, HbE, HbS, HbF et HbA des hémoglobines. L'analyse par électrophorèse d'un échantillon biologique permet de séparer les différentes protéines, en particulier les différentes hémoglobines, présentes dans l'échantillon, et de déterminer la quantité et/ou la proportion d'une ou plusieurs protéine(s) d'intérêt dans l'échantillon biologique. En électrophorèse capillaire, dans un capillaire rempli d'un électrolyte, les protéines d'un échantillon biologique migrent sous l'effet d'un champ électrique de l'anode vers la cathode, en fonction de leur masse et de leur charge. Elles forment ainsi un profil de migration électrophorétique comportant une série de pics (également appelés fractions), chacun d'entre eux correspondant à une ou plusieurs protéines. Ainsi, des hémoglobines anormales telles que HbS, HbC et HbE présentent une mobilité électrophorétique différente de celle de HbA. De plus, de par le résidu ose lié sur l'acide aminé N-terminal des chaînes bêta, l'HbA₁ a un point isoélectrique réduit et, par conséquent, une charge électrique légèrement différente de celle des autres hémoglobines de type HbA₀. Ainsi, dans un champ électrophorétique ou dans une résine échangeuse d'ions, l'HbA₁ présente une vitesse de migration différente de celle de l'HbA₀, ce qui permet de séparer l'HbA₁ de l'HbA₀, qui est différemment chargée.

L'intérêt de l'électrophorèse capillaire réside également dans le fait que seules de très petites quantités d'échantillon biologique à analyser sont nécessaires. De plus, la séparation par cette technique peut être très rapide, dans la mesure où de forts voltages peuvent être utilisés sans que l'échantillon ne s'échauffe trop lors de la séparation.

L'analyse en focalisation isoélectrique (Molteni, 1994 ; Hempe, 1994) est réalisée en capillaires revêtus. Pour le revêtement (« coating »), on utilise une solution de methylcellulose. Le catholyte utilisé est la soude (NaOH) et l'anolyte de l'acide phosphorique (H₃PO₄). Grâce à l'utilisation d'ampholytes, cette analyse permet de séparer les différents variants de l'hémoglobine ainsi que l'HbA_{1c} dont le pic est très proche de celui de l'HbA (ΔpI < 0.10). Bien que cette méthode fonctionne bien, elle n'est pas simple à mettre en œuvre en routine et nécessite une grande précision, notamment au niveau de la gamme de pH des ampholytes (le point isoélectrique (pI) de HbA_{1c} étant très proche à celui de HbA₀).

L'analyse en solution libre faisant intervenir un anticorps anti-HbA_{1c} (US 5,431,793) est réalisée en tampon borate, à pH basique (pH 8 à 10), sur des échantillons soumis à une réaction avec un anticorps monoclonal anti-HbA_{1c}. Plus précisément, un premier électrophorégramme est réalisé sur l'échantillon de travail sans réaction préalable avec un anticorps anti-HbA_{1c}. Un seul pic est obtenu, d'aire x, contenant toutes les hémoglobines (dont l'HbA_{1c}). Un deuxième électrophorégramme est obtenu par l'analyse de l'échantillon de travail, préalablement mis en contact avec l'anticorps anti-HbA_{1c}. On sépare ainsi l'anticorps anti-HbA_{1c} non lié, le complexe anticorps anti-HbA1_{c}/HbA_{1c} et les hémoglobines non complexées par l'anticorps. La quantité de HbA_{1c} est alors déterminée par différence d'aire de pics, entre le pic du premier électrophérogramme et celui du second relatif aux hémoglobines non complexées par l'anticorps anti-HbA_{1c}, d'aire y, soit une quantité x-y. Cette méthode s'avère ainsi longue et compliquée à mettre en œuvre.

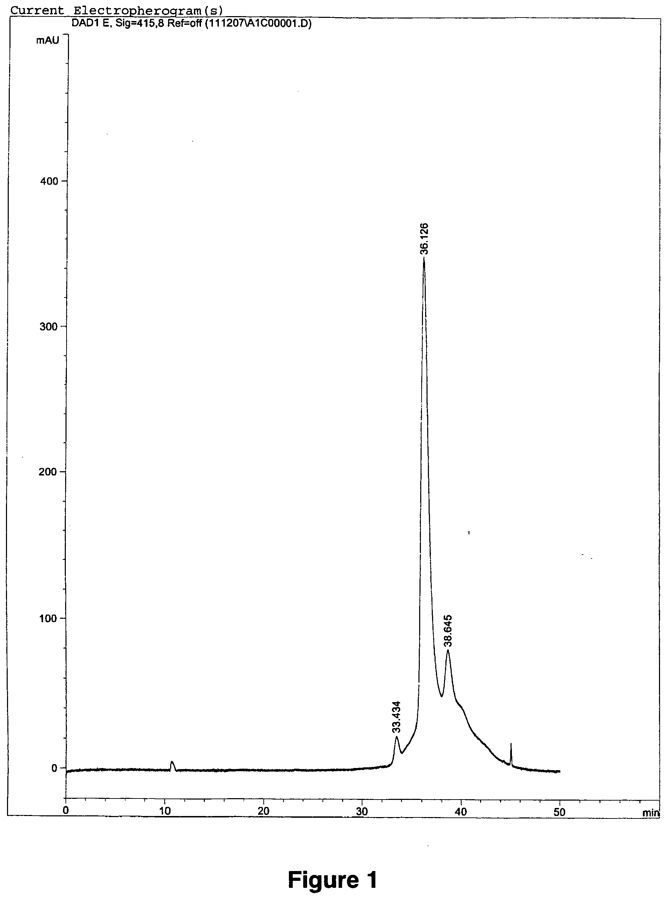

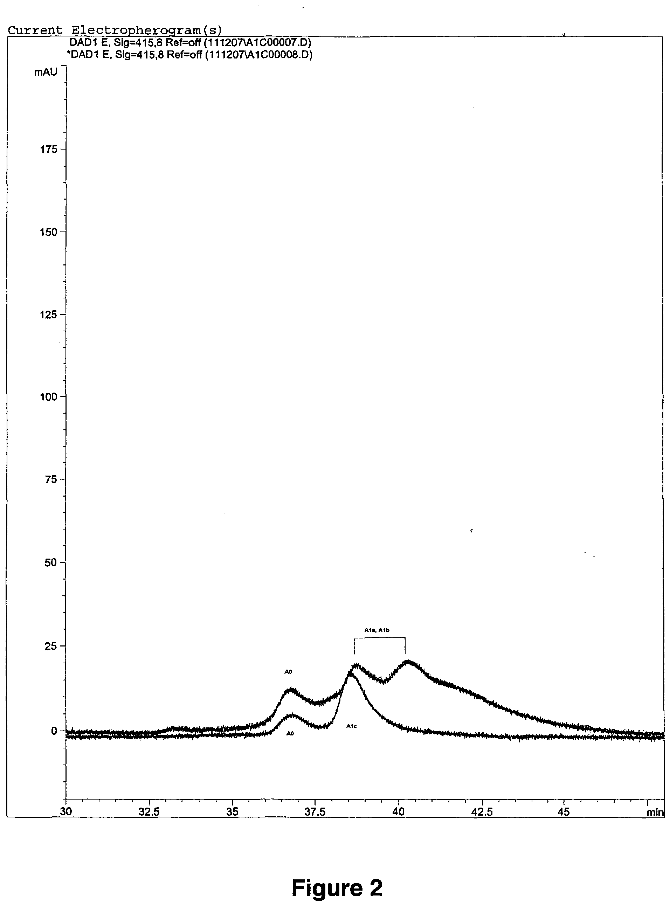

La méthode d'analyse en solution libre, en capillaire nu (US 5,599,433 et WO 96/22524) fait intervenir un agent complexant le sucre lié à l'hémoglobine (le borate) et un tampon zwitterionique (le CAPS) à pH basique (pH 9 à 12). Le profil obtenu (présenté en figure 1 de la présente demande) dans les conditions décrites montre une séparation assez faible des pics de HbA₁ (décrit comme étant le pic de HbA_{1c} dans le brevet US 5,599,433 et WO 96/22524) et HbA₀ (voir exemple 1). Par ailleurs, l'analyse de fractions mineures purifiées laisse apparaître une co-migration des fractions HbA_{1a,b} et HbA_{1c}, interférant dans le résultat final du dosage (voir exemple 2 et figure 2 de la présente demande). En fait, cette technique sépare les fractions HbA0 et HbA1, mais ne sépare pas les fractions HbA1 entre elles.

La technique d'analyse en capillaire avec double revêtement dynamique (EP 0 733 900 A2 ; US 5,611,903) est utilisée dans le seul test basé sur l'électrophorèse capillaire qui soit commercialisé (kit Analis CEofix™ HbA_{1c}). Elle consiste en un premier lavage du capillaire par un «initiateur» contenant un polycation (en solution à pH 4.6), produisant un revêtement de la paroi du capillaire par ce polycation. Un second lavage est ensuite réalisé avec le tampon d'analyse contenant un polyanion (pH 4.6), qui a pour effet d'ajouter une seconde couche de revêtement par interaction avec le polycation. La quantité de charges électriques négatives alors présente sur la paroi interne du capillaire est encore plus élevée que sur un capillaire nu, d'où un flux électroosmotique encore plus important. La résolution obtenue entre les différentes formes d'hémoglobine analysées (dont les hémoglobines A1 glyquées) est satisfaisante, avec des durées d'analyse courtes. D'un point de vue pratique, ce double revêtement doit être refait entre chaque analyse selon une procédure précise fournie par le fabricant du kit mais qui n'a été décrite que pour un appareil mono-capillaire (P/ACE, Beckman), non adapté à des analyses de routine.

Il subsiste donc un besoin pour des réactifs et une méthode simple à mettre en œuvre permettant de séparer efficacement des hémoglobines glyquées, en particulier l'HbA_{1c}, des autres hémoglobines, variants, interférents (formes labiles, acétylées, carbamylées) et des autres fractions mineures (notamment HbA₁ₐ et HbA_{1b}) présentes dans un échantillon biologique contenant d'autres hémoglobines. Idéalement, une telle méthode permettrait d'obtenir un dosage semi-quantitatif ou quantitatif d'hémoglobines glyquées, en particulier de l'HbA_{1c}, directement à partir du profil électrophorétique obtenu.

La présente invention propose une méthode d'électrophorèse capillaire alternative, telle qu'elle est défini dans le revendication 1, qui permet de réaliser une analyse semi-quantitative ou quantitative d'une ou plusieurs hémoglobine(s) glyquée(s) comportant au moins une chaîne de globine comportant un résidu glucose lié à l'acide aminé se trouvant en position N-terminale, contenue(s) dans un échantillon biologique comportant notamment d'autres hémoglobines (glyquées et/ou non glyquées). Ladite méthode de l'invention exploite à la fois les caractéristiques structurelles des molécules de glucose liées à ces hémoglobines glyquées et les différences de charge existant entre les différentes hémoglobines de l'échantillon.

Les inventeurs ont montré qu'en utilisant une composition tampon particulière, il est possible d'obtenir une séparation largement améliorée des hémoglobine(s) glyquée(s) comportant un résidu glucose lié à l'acide aminé se trouvant en position N-terminale sur au moins une chaîne de globine, et selon l'invention sur les chaînes bêta, et plus particulièrement une séparation largement améliorée de l'HbA_{1c}.

L'un des avantages de la méthode de l'invention est de permettre, du fait de l'utilisation de ladite composition tampon, un déplacement du pic d'électrophorèse correspondant à un ou plusieurs types d'hémoglobine(s) glyquée(s) d'intérêt (un ou plusieurs types d'hémoglobine(s) comportant un résidu glucose lié à l'acide aminé se trouvant en position N-terminale sur au moins une chaîne de globine, par exemple l'HbA_{1c}) lorsqu'elle est présente dans un échantillon biologique par rapport à la position du pic qui serait obtenue sans la composition tampon. Ce déplacement n'interfère pas avec la séparation des autres protéines, en particulier des autres hémoglobines (glyquées et/ou non glyquées) de l'échantillon. Ceci permet notamment de séparer l'HbA_{1c} d'autres HbA₁ mineures (notamment HbA₁ₐ et HbA_{1b}). En conséquence, cette méthode permet une lecture fiable des résultats de l'analyse et un dosage précis d'une ou plusieurs hémoglobine(s) glyquée(s) d'intérêt comportant un ou plusieurs résidu(s) glucose avec un résidu glucose lié à l'acide aminé se trouvant en position N-terminale sur au moins une chaîne de globine (par exemple l'hémoglobine A_{1c}).

La méthode de l'invention constitue donc une méthode de choix en vue de l'établissement d'un diagnostic ou du suivi de patients diabétiques notamment pour évaluer l'efficacité d'un traitement anti-diabétique.

Ainsi la présente demande décrit une méthode d'analyse, par électrophorèse capillaire, d'hémoglobines glyquées (une ou plusieurs hémoglobine(s) glyquée(s)) comportant un ou plusieurs résidu(s) glucose, contenues dans un échantillon biologique comprenant une ou plusieurs hémoglobine(s), ladite méthode comprenant la mise en œuvre d'une composition tampon comprenant au moins un composé capable d'une part de complexer spécifiquement des résidus glucose (un ou plusieurs résidu(s)) d'hémoglobines glyquées (une ou plusieurs hémoglobine(s) glyquée(s)) de l'échantillon biologique et d'autre part d'apporter à ces hémoglobines glyquées plusieurs charge(s) électrique(s) négative(s) à pH alcalin (c'est-à-dire à un pH supérieur 7).

Par « plusieurs », on entend, au sens de la présente demande, au moins deux, c'est-à-dire deux ou plus de deux, par exemple, deux, trois, quatre, cinq, six, sept, huit, neuf, dix ou plus de dix.

Par « au moins un(e)», on entend, au sens de la présente demande, un(e) ou plusieurs.

Le terme « hémoglobine », englobe, au sens de la présente demande, toute forme ou fraction d'hémoglobine (y compris les fractions mineures), qu'elle soit normale ou anormale, ainsi que les nombreux variants de ces hémoglobines (il existe au moins 1000 variants décrits de l'hémoglobine).

Par « hémoglobine glyquée », on entend, dans la présente demande, toute hémoglobine modifiée par la fixation d'un ou plusieurs sucre(s), en particulier d'un ou plusieurs glucose, glucose-6-phosphate, fructose 1-6 di-phosphate ou pyruvate, sur une ou plusieurs chaîne(s) de globine (quelle(s) que soit (soient) la ou les chaîne(s) de globine). Le(s) sucre(s) peut (peuvent) être fixés sur un acide aminé se trouvant en position N-terminale sur une chaîne de globine et/ou sur un acide aminé ayant un groupe amino libre (la lysine par exemple).

Le terme « chaîne de globine » désigne, dans la présente demande, n'importe quelle chaîne de globine connue de l'homme du métier, en particulier une chaîne choisie parmi les chaînes alpha (α), bêta (β), delta (δ) et gamma (γ), qu'elles soient normales ou anormales ou encore un variant de l'une de ces chaînes. Selon l'invention, ladite « chaîne de globine » est une chaîne bêta, par exemple une chaîne bêta d'une hémoglobine A₁, en particulier A_{1c}, S ou C.

Lorsque qu'il est fait référence à l'hémoglobine glyquée, il faut entendre que cette mention s'applique en particulier à chaque type particulier d'hémoglobine glyquée cité dans la présente demande.

Sauf indication contraire, chaque mode de réalisation indiqué dans cette demande s'applique indépendamment et/ou en combinaison avec un quelconque ou plusieurs des autres modes de réalisation décrits.

La méthode d'analyse par électrophorèse capillaire de l'invention s'applique à un échantillon biologique comprenant au moins une hémoglobine glyquée comportant un ou plusieurs résidu(s) glucose et comportant nécessairement, sur une ou plusieurs chaîne(s) de globine bêta, un résidu glucose lié à l'acide aminé se trouvant en position N-terminale. Ces hémoglobines sont aussi dénommées « hémoglobines glyquées en position N-terminale » dans la présente demande.

Ladite méthode permet de séparer une ou plusieurs hémoglobine(s) glyquée(s) en position N-terminale des autres hémoglobines glyquées (c'est-à-dire des hémoglobines glyquées dont les chaînes de globine sont dépourvues de résidu glucose lié à l'acide aminé se trouvant en position N-terminale) ou des hémoglobines non glyquées, qui sont éventuellement présentes dans l'échantillon biologique analysé.

Selon un mode de réalisation particulier, l'acide aminé se trouvant en position N-terminale sur les chaîne(s) bêta est la valine.

La méthode d'analyse de l'invention est, dans un mode de réalisation préféré, destinée à analyser un échantillon biologique comprenant de l'hémoglobine A_{1c}. Elle est en particulier appropriée pour séparer l'hémoglobine A_{1c} d'autres ou des autres hémoglobines (glyquées différemment ou non glyquées) éventuellement présentes dans l'échantillon biologique analysé, et en particulier d'autres fractions mineures, par exemple de l'HbA₁ₐ et de l'HbA_{1b}.

Selon un mode de réalisation particulier, la méthode d'analyse de l'invention permet de séparer l'hémoglobine S_{1c} et/ou l'hémoglobine C_{1c}, des autres hémoglobines (glyquées différemment ou non glyquées) éventuellement présentes dans l'échantillon biologique analysé.

Par « composition tampon », on entend une composition, en particulier une solution, qui conserve approximativement le même pH malgré l'addition de petites quantités d'un acide ou d'une base ou malgré une dilution.

Le terme « complexer » ou « complexation » est utilisé dans la présente demande pour signifier qu'une association (chimique ou simplement physique) a lieu entre deux entités, en particulier entre deux molécules (par exemple une petite molécule et une macromolécule) via des groupements fonctionnels.

Dans un mode de réalisation préféré, l'une de ces deux entités (le composé capable de complexer des résidus glucose) s'associe avec (par exemple réagit avec) un groupement cis-diol, en particulier avec deux groupements hydroxyle vicinaux, d'un résidu glucose de l'autre entité (une hémoglobine glyquée).

Comme montré dans la partie « exemples », « complexer spécifiquement le(s) résidu(s) glucose d'une ou plusieurs hémoglobine(s) glyquée(s) et leur apporter des charges négatives à pH alcalin» signifie, dans la présente demande, que le composé utilisé dans une composition tampon dans le cadre d'une méthode selon invention permet de déplacer le pic de migration électrophorétique correspondant à une hémoglobine glyquée en position N-terminale par du glucose, en dehors de la zone de migration électrophorétique correspondant à des hémoglobines glyquées en position N-terminale par un autre sucre (par exemple du glucose-6-phosphate, du fructose 1-6 di-phosphate, ou du pyruvate), et de préférence en dehors de la zone des pics de migration électrophorétique correspondant aux autres hémoglobines de l'échantillon. Ainsi, les fractions mineures qui ne comportent pas de résidu glucose lié à l'acide aminé N-terminal des chaînes bêta, en particulier les fractions A₁ₐ et A_{1b}, n'interfèrent pas avec l'analyse de l'HbA_{1c} selon la méthode selon l'invention.

A titre d'exemple, la capacité d'un composé à complexer spécifiquement des résidus glucose d'une ou plusieurs hémoglobine(s) glyquée(s) et à apporter des charges négatives à pH alcalin au sens de l'invention peut être mise en évidence par le test suivant : un échantillon de référence (par exemple « HbA₁ₐ, HbA_{1b} mixture », « A_{1c} » et « A₀ » de Exocell, USA), contenant différentes fractions d'hémoglobine glyquée purifiées et en particulier les fractions A₀ et A_{1c} ou les fractions A₀, A_{1b} et A₁ₐ, est introduit dans un capillaire d'électrophorèse capillaire contenant la composition tampon suivante : 200mM de CHES, 20mM de putrescine, entre 10 et 120 mM (par exemple 30 mM ou 50mM) de composé à tester, le cas échéant 2.5 g/l de chlorure de sodium, de l'eau, et le cas échéant, une base pour ajuster le pH à une valeur supérieure à 9, par exemple à 9,4. Une électrophorèse capillaire en solution libre en capillaire nu est alors réalisée, par exemple sur un appareil Capillarys® (Sebia), à une longueur d'onde de 415nm. On étudie ensuite le profil électrophorétique obtenu; si la séparation est parfaite entre le pic correspondant à HbA_{1c} et le pic correspondant à HbAo (ou, en fonction du type d'échantillon utilisé, entre le pic correspondant à HbA_{1c} et les pics correspondant à HbA₀, HbA_{1b} et HbA₁ₐ), alors le composé testé est considéré comme étant capable de complexer spécifiquement des résidus glucose et d'apporter des charges négatives à pH alcalin au sens de l'invention. Au contraire, si le pic correspondant à HbA_{1c} et celui qui correspond à HbA_{1b} (ou, en fonction du type d'échantillon utilisé, le pic correspondant à HbA_{1c} ou celui qui correspond à HbA₀, HbA_{1b} ou HbA₁ₐ) se superposent ou se chevauchent alors le composé testé n'est pas considéré comme étant approprié pour mettre en œuvre l'invention.

La concentration du composé à tester n'est donnée qu'à titre indicatif dans le test indiqué ci-dessus; si une concentration de 10 à 120 mM de composé à tester ne permet pas d'obtenir une séparation suffisante entre le pic correspondant à HbA_{1c} et le pic correspondant aux autres hémoglobines de l'échantillon dans le cadre du test indiqué ci-dessus, il est possible de sortir de cette gamme de concentration afin d'obtenir une séparation optimale du pic correspondant à HbA_{1c}.

Tout composé chimique (c'est-à-dire généralement une molécule organique), tout anticorps, polypeptide, peptide, ou tout autre composé capable de complexer spécifiquement des résidus glucose et d'apporter des charges négatives à pH alcalin au sens de la présente description peut ainsi être utilisé. Un anticorps approprié peut être généré par toute méthode connue de l'homme du métier et peut être, par exemple, un anticorps polyclonal ou monoclonal, un anticorps chimérique, ou un fragment d'anticorps, par exemple un fragment Fab. Selon l'invention, un composé présente les caractéristiques définies dans la revendication 1.

Comme illustré dans la partie « exemples », l'acide borique n'est pas un composé chimique capable de complexer spécifiquement des résidus glucose et d'apporter des charges négatives à pH alcalin au sens de la présente invention, car il ne permet pas de réaliser une analyse fiable et en particulier un dosage fiable d'hémoglobines glyquées et notamment de l'HbA_{1c}. Des exemples de composés chimiques appropriés sont définis plus précisément ci-après.

Chaque résidu de glucose complexé avec le composé capable de complexer spécifiquement des résidus glucose interagit avec une molécule de composé différente.

Selon un mode de réalisation particulier, lorsqu'il est présent en quantité suffisante dans une composition tampon employée dans une méthode selon l'invention, ledit composé est capable de complexer spécifiquement le(s) résidu(s) glucose N-terminaux d'une hémoglobine glyquée c'est à dire de complexer spécifiquement, pour chaque chaîne de globine bêta comportant un résidu glucose lié à l'acide aminé se trouvant en position N-terminale, ledit résidu glucose. Ledit composé est ainsi capable de complexer spécifiquement chaque résidu glucose se trouvant lié à la valine en position N-terminale sur les chaînes bêta d'une hémoglobine A_{1c} ou encore d'une hémoglobine S_{1c} ou C_{1c}.

Selon un mode de réalisation particulier, ledit composé est capable, lorsqu'il est présent en quantité suffisante dans une composition tampon employée dans une méthode selon l'invention, de complexer spécifiquement tous les résidus glucose des hémoglobines glyquées en position N-terminale par un glucose.

Selon un mode de réalisation particulier, ledit composé est capable, lorsqu'il est présent en quantité suffisante dans une composition tampon employée dans une méthode selon l'invention, de complexer spécifiquement tous les résidus glucose d'une hémoglobine, quelle que soit leur position sur la ou les chaîne(s) de globine. Ainsi, lorsque ledit composé est présent en quantité suffisante dans une composition tampon employée dans une méthode selon l'invention, une molécule d'hémoglobine comportant un nombre x de résidus glucose peut fixer un nombre x de molécules dudit composé.

En se complexant spécifiquement avec un ou plusieurs résidu(s) glucose d'hémoglobines glyquées d'un échantillon biologique, ledit composé apporte à cette ou ces hémoglobine(s) glyquée(s) plusieurs charges électriques négatives à pH alcalin, c'est-à-dire permet de surcharger cette (ces) hémoglobine(s) glyquée(s) en charges électriques négatives à pH alcalin.

Ledit composé apporte plusieurs charges électriques négatives à pH alcalin à chaque résidu glucose ainsi complexé d'une même hémoglobine. De par cet apport de charges électriques négatives à pH alcalin, les hémoglobines comportant au moins un résidu glucose voient leur mobilité électrophorétique se modifier sous l'effet de leur complexation avec ledit composé. Cela se manifeste, sur un profil électrophorétique, par le déplacement du ou des pics d'électrophorèse correspondant aux hémoglobines glyquées comportant un ou plusieurs résidu(s) glucose qui sont présentes dans l'échantillon biologique analysé.

Toutes les hémoglobines comportant au moins un résidu glucose qui sont présentes dans l'échantillon biologique forment un complexe avec le composé complexant le glucose et apportant des charges électriques négatives à pH alcalin (à condition, bien entendu, que ledit composé soit présent en quantité suffisante pour complexer chacune de ces hémoglobines). Cependant, les différentes hémoglobines et en particulier les différentes hémoglobines comportant au moins un résidu de glucose ont toutes à l'origine un point isoélectrique différent (de par la nature de leurs chaînes de globine, le nombre et la position du ou des résidu(s) glucose sur ces chaînes de globines et le nombre, la nature et la position des autres sucres éventuellement liés sur ces hémoglobines). Compte tenu des charges électriques négatives à pH alcalin apportées par la complexation de chaque résidu glucose avec ledit composé, la différence de charge entre ces différentes hémoglobines est encore plus marquée lorsqu'elles sont sous forme complexée avec ledit composé.

Les hémoglobines glyquées qui comportent un résidu glucose lié sur l'acide aminé se trouvant en position N-terminale sur au moins une de leurs chaînes de globine bêta possèdent de ce fait au moins un glucose de plus que les autres hémoglobines (glyquées différemment ou non glyquées). En particulier l'hémoglobine A_{1c} possède sur les chaînes bêta au moins un glucose de plus que les autres fractions mineures de l'hémoglobine A₁. Par conséquent, lorsqu'elles sont complexées dans le cadre d'une méthode selon l'invention, ces hémoglobines glyquées en position N-terminale sont chargées plus fortement en charges électriques négatives à pH alcalin par ledit composé que les autres hémoglobines (les hémoglobines glyquées ne comportant pas de résidu glucose lié sur l'acide aminé N-terminal des chaînes de globine ou les hémoglobines non glyquées).

En augmentant ainsi la différence de mobilité électrophorétique existant entre les différentes hémoglobines comportant du glucose et/ou entre une ou plusieurs hémoglobine(s) comportant du glucose et les autres hémoglobines présentes dans l'échantillon biologique analysé, on obtient une meilleure séparation entre une ou plusieurs hémoglobine(s) glyquée(s) en position N-terminale et la ou les autre(s) hémoglobine(s) éventuellement présente(s) dans ledit échantillon, en particulier entre l'hémoglobine A_{1c} et les autres fractions mineures de l'hémoglobine A₁ éventuellement présente(s) dans ledit échantillon.

Ainsi, la méthode de l'invention permet de séparer efficacement des hémoglobines glyquées en position N-terminale, et en particulier l'HbA_{1c}, des autres hémoglobines, de certains variants et des autres fractions mineures présentes dans un échantillon biologique contenant d'autres hémoglobines. En outre, des interférents classiques tels que les formes labiles, acétylées ou carbamylées n'interfèrent pas avec le dosage de l'HbA_{1c} selon la méthode de l'invention. La méthode de l'invention permet en particulier d'éviter les interférences qui pourraient résulter, en l'absence de charges électriques négatives à pH alcalin ajoutées par la complexation, de la co-migration de l'hémoglobine A_{1c} et des autres fractions mineures de l'hémoglobine A₁ (dont l'HbA₁ₐ et l'HbA_{1b}) présentes dans l'échantillon biologique, lors de l'étape de séparation électrophorétique.

La ou les hémoglobine(s) glyquée(s) en position N-terminale ainsi séparée(s) peut (peuvent) ensuite être dosée(s) et ce, en présence d'autres ou des autres hémoglobines éventuellement présentes dans l'échantillon biologique analysé. On peut donc doser, par exemple, l'HbA_{1c} en présence d'autres fractions mineures de l'hémoglobine A₁ et en particulier en présence de HbA₁ₐ et/ou HbA_{1b}, sans que ces autres fractions mineures présentes dans l'échantillon biologique analysé n'interfèrent avec ce dosage.

Le terme « doser » ou « dosage » signifie, dans la présente demande, que l'on détermine la quantité de l'hémoglobine ou des hémoglobines d'intérêt en présence éventuellement d'une ou plusieurs autre(s) hémoglobine(s) de l'échantillon biologique analysé et/ou la proportion de ladite ou desdites hémoglobine(s) d'intérêt par rapport à la quantité totale d'hémoglobine ou par rapport à la quantité de certaines hémoglobines présentes dans ledit échantillon.

Le dosage réalisé dans le cadre de l'invention peut être semi-quantitatif ; on mesure alors le pourcentage d'une hémoglobine donnée par rapport à la quantité d'une autre hémoglobine ou d'autres hémoglobine(s) présente(s) dans ledit échantillon. Ainsi, dans le cas de l'HbA_{1c}, on mesure alors généralement le pourcentage d'HbA_{1c} par rapport à la quantité d'une ou plusieurs autre(s) hémoglobine(s) A ; on divise en général l'aire du pic électrophorétique correspondant à HbA_{1c} par l'aire du pic correspondant à HbAₒ ou bien par la somme (aire du pic correspondant à HbA_{1c} + aire du pic correspondant à HbAₒ), par la somme (aire de tous les pics d'HbA) ou bien par la somme (aire du pic correspondant à HbA_{1c} + aire du pic correspondant à HbAₒ + aire du pic correspondant à HbA₂), ou encore par la surface totale du profil électrophorétique.

Il peut également s'agir d'un dosage quantitatif ; les résultats sont alors exprimés en millimoles (mmoles) d'une hémoglobine donnée par mole d'une autre hémoglobine ou d'autres hemoglobine(s) présente(s) dans ledit échantillon, par exemple en mmoles d'HbA1c par mole d'HbA.

Dans le cadre de la méthode selon l'invention, le composé complexant spécifiquement des résidus glucose d'hémoglobines glyquées et apportant à ces hémoglobines glyquées des charges négatives à pH alcalin comprend deux ou plus de deux groupements fonctionnels.

L'un au moins de ces groupements fonctionnels complexe spécifiquement un résidu glucose lié à l'acide aminé se trouvant en position N-terminale sur une chaîne de globine en interagissant par exemple avec un groupement cis-diol, en particulier avec deux groupements hydroxyle vicinaux, d'un résidu glucose. A titre d'exemple, ce(s) groupement(s) peut (peuvent) être un groupement boronate, en particulier un groupement boronate tel que défini dans la présente demande.

Ce(s) groupement(s) complexant le glucose, peut (peuvent) apporter une charge électrique négative à pH alcalin par résidu glucose complexé, soit une ou plusieurs charge(s) négative(s) à pH alcalin par molécule d'hémoglobine complexée (une charge négative si la molécule d'hémoglobine ne comporte qu'un résidu glucose et n charges négatives si la molécule d'hémoglobine comporte n résidus glucose).

L'autre, un des autres, ou les autres groupement(s) fonctionnel(s) dudit composé (c'est-à-dire le(s) groupement(s) fonctionnel(s) qui ne complexe(nt) pas le glucose) apportent quant à eux une ou plusieurs charge(s) électriques(s) négative(s) à pH alcalin pour chaque résidu de glucose complexé d'une molécule de ladite hémoglobine.

Ainsi, dans le cadre de la méthode selon l'invention, chaque molécule de composé complexant spécifiquement des résidus glucose comporte au moins un groupement fonctionnel capable de complexer un résidu glucose N-terminal d'une hémoglobine glyquée en position N-terminale et au moins un groupement fonctionnel qui ne complexe pas le glucose mais permet de surcharger cette hémoglobine glyquée en charges négatives à pH alcalin car il apporte une ou plusieurs charge(s) négative(s) supplémentaires à pH alcalin à cette hémoglobine glyquée . Le groupement fonctionnel capable de complexer le glucose peut également apporter une charge électrique négative à pH alcalin.

Dans le cadre de la méthode selon l'invention, le composé complexant spécifiquement des résidus glucose et apportant des charges négatives à pH alcalin (plus précisément chaque molécule de ce composé) apporte, pour chaque résidu de glucose complexé, plusieurs charges électriques négatives à pH alcalin à une molécule d'hémoglobine glyquée en position N-terminale. De préférence, il apporte pour chaque résidu de glucose complexé, au moins deux (en particulier deux, trois, quatre, cinq ou six) charges électriques négatives à pH alcalin à une molécule d'hémoglobine glyquée.

Selon un mode de réalisation particulier de l'invention, le groupement fonctionnel ou au moins un des groupements fonctionnels complexant spécifiquement des résidus glucose est négativement chargé à pH alcalin.

Selon un mode de réalisation particulier de l'invention, le composé capable de complexer spécifiquement des résidus glucose d'hémoglobines glyquées et de leur apporter des charges négatives à pH alcalin comporte un ou plusieurs groupements ionisables (en particulier anionisables) à pH alcalin. Ces groupements ionisables peuvent être de différents types. Par exemple, le composé peut comporter un ou plusieurs carboxylate(s), carboxyle(s), sulfonate(s) et/ou sulfonyle(s). Ledit composé peut notamment être un acide polycarboxylique, en particulier un acide dicarboxylique ou tricarboxylique. Ledit composé peut également être un acide polysulphonique en particulier un acide disulphonique ou trisulphonique.

Selon un mode de réalisation particulier de l'invention, le groupement, un des groupements ou les groupements apportant une ou plusieurs charge(s) électriques(s) négative(s) à pH alcalin pour chaque résidu de glucose complexé d'une molécule d'hémoglobine glyquée comprend (comprennent) ou consiste(nt) en un ou plusieurs groupement(s) ionisable(s) à pH alcalin tels que définis dans la présente demande, et en particulier un ou plusieurs (notamment deux ou trois) groupement(s) carboxylate, carboxyle, sulfonate et/ou sulfonyle.

Selon un mode de réalisation particulier de l'invention, le groupement, un des groupements ou les groupements complexant spécifiquement des résidus glucose comprend (comprennent) ou consiste(nt) en un ou plusieurs groupement(s) boronate.

Par « groupement boronate », on entend, dans la présente demande, un groupement de formule : 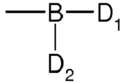 dans lequel D₁ et D₂ sont choisis, indépendamment l'un de l'autre, parmi un groupement hydroxyle et un groupement susceptible d'être hydrolysé pour donner un groupement hydroxyle en solution aqueuse, en particulier à un pH alcalin.

Dans un mode de réalisation particulier de l'invention, ledit groupement boronate est le groupement -B(OH)₂, (également appelé groupement boronyle) ou une forme ionisée, en particulier -B(OH)⁻₃.

Dans un mode de réalisation particulier de l'invention, le composé capable de complexer spécifiquement des résidus glucose d'hémoglobines glyquées et de leur apporter des charges négatives à pH alcalin est
(i) un composé boronate, de formule générale RB(OH)₂ (composé également appelé acide boronique), ou de formule générale RB(OH)⁻₃, dont le groupement R comprend au moins un aryle et/ou un alkyle (linéaire, ramifié ou cyclique) et/ou un aralkyle et/ou une combinaison de ceux-ci, et ledit groupement R apporte une ou plusieurs charge(s) électriques(s) négative(s) à pH alcalin pour chaque résidu de glucose complexé avec le groupement boronate ; ou
(ii) un sel de ce composé boronate.

Ainsi, à pH alcalin, outre la charge électrique négative à pH alcalin apportée par le groupement boronyle ou boronate du composé boronate ou de son sel, le groupement R apporte une ou plusieurs charge(s) électrique(s) négative(s) supplémentaires pour chaque résidu de glucose complexé avec le groupement boronyle ou boronate.

Par « composé de formule générale RB(OH)₂ », on entend également, au sens de la présente demande, toute forme ionique en équilibre avec celle-ci, notamment, RB(OH)⁻₃, ou toute forme susceptible d'être en équilibre avec celle-ci en fonction des conditions du milieu.

Un « sel » au sens de la présente demande désigne tout sel et en particulier un sel de sodium, de lithium ou de potassium.

Le groupement R du composé boronate peut également inclure d'autres fonctions et/ou hétéroatomes, outre la ou les fonctions aryle, alkyle, aralkyle ou une de leurs combinaisons.

Selon un mode de réalisation particulier, le groupement R apporte deux ou plus de deux (de préférence deux) charges électriques négatives à pH alcalin pour chaque résidu de glucose complexé avec le groupement boronyle ou boronate du composé boronate de l'invention ou de son sel.

Selon un mode de réalisation particulier, le groupement R consiste en un ou plusieurs aryle(s), alkyle(s) (linéaire(s), ramifié(s) ou cyclique(s)) et/ou aralkyle(s) et/ou une combinaison de ceux-ci.

Selon un mode de réalisation particulier, le ou les aryle(s), alkyle(s), aralkyle(s) ou les autres groupements fonctionnels et/ou hétéroatomes ou leurs combinaisons présent(s) dans le groupement R du composé boronate peut (peuvent) être substitué(s).

Le terme « substitué » tel qu'utilisé dans la présente demande peut signifier monosubstitué ou au contraire polysubstitué, notamment di-, tri-, tetra-, penta- ou hexa- substitué.

Les substituants peuvent être notamment des groupements ionisables (en particulier anionisables) à pH alcalin tels que définis dans la présente demande. Le groupement R peut également inclure un ou des hétéroatomes, ou d'autres groupes fonctionnels.

Selon un mode de réalisation particulier de l'invention, le groupement R du composé boronate apporte, pour chaque résidu de glucose complexé, une ou plusieurs charge(s) électrique(s) négatives à pH alcalin à une molécule d'hémoglobine glyquée. De préférence, il apporte pour chaque résidu de glucose complexé, une, deux, trois ou quatre charges électriques négatives à pH alcalin à une molécule d'hémoglobine glyquée.

Un composé boronate tel que défini ci-dessus inclut notamment des acides boroniques, et en particulier des acides phénylboroniques.

Selon un mode de réalisation particulier de l'invention, le composé capable de complexer spécifiquement des résidus glucose d'hémoglobines glyquées et de leur apporter des charges négatives à pH alcalin est un phénylboronate polysubstitué, en particulier un phénylboronate disubstitué, par exemple par des groupements carboxyle(s) et/ou sulfonyle(s).

Selon un mode de réalisation particulier de l'invention, le groupement R du composé boronate ou de son sel est un diacide, en particulier un acide dicarboxylique.Selon un mode de réalisation particulier de l'invention, le composé boronate est un acide diCarboxyPhenylBoronique, de préférence choisi parmi l'acide 3,5-diCarboxyPhenylBoronique et l'acide 3,4-diCarboxyPhenylBoronique.

Ainsi, à titre d'exemple, on peut utiliser, à titre de composé boronate, l'acide 3,5-diCarboxyPhenylBoronique (3,5-dCPBA). Ce composé est disponible dans le commerce, notamment auprès de la société Combi-blocks Inc. (San Diego, USA), sous le nom commercial 3,5-dicarboxyphenylboronic acid et auprès de la société Apollo Scientific Ltd (Cheshire, Royaume-Uni), sous le nom commercial 3,5-dicarboxybenzeneboronic acid.

Dans la composition tampon utilisée dans le cadre d'une méthode selon l'invention, la concentration du composé capable de complexer spécifiquement des résidus glucose d'hémoglobines glyquées et de leur apporter des charges négatives à pH alcalin est généralement en excès stoechiométrique par rapport à la quantité totale de protéines, en particulier par rapport à la quantité totale de toutes les hémoglobines présentes dans l'échantillon biologique ou par rapport à la quantité totale de toutes les hémoglobines comportant du glucose présentes dans l'échantillon biologique. Ainsi, la quantité de ce composé dans la composition tampon est supérieure à la quantité nécessaire pour que tous les résidus glucose des hémoglobines présentes dans l'échantillon soient complexés par ledit composé lorsqu'on dilue l'échantillon ou une partie aliquote de cet échantillon dans la composition tampon. Ainsi, pour chaque molécule d'hémoglobine glyquée comportant du glucose présente dans l'échantillon biologique analysé, il y a au moins autant de molécules de ce composé dans la composition tampon que de résidus glucose présents dans cette molécule d'hémoglobine glyquée. Ceci permet d'obtenir une séparation complète de l'hémoglobine glyquée ou des hémoglobines glyquées d'intérêt des autres hémoglobines également présentes dans l'échantillon biologique analysé.

Selon un mode de réalisation particulier de l'invention, la concentration, dans la composition tampon, du composé capable de complexer spécifiquement des résidus glucose d'hémoglobines glyquées et de leur apporter des charges négatives à pH alcalin est comprise entre 0,10 et 100 mM, de préférence entre 10 et 60 mM, et plus préférablement entre 20 et 50 mM, par exemple 30 mM ou 50mM.

L'expression « compris(e) entre x et y » signifie, dans la présente demande que les bornes x et y sont incluses dans la gamme de valeur x-y indiquée.

Selon un mode de réalisation particulier, la composition tampon comprend en outre:
- un composé tampon ayant pKa compris entre 8,0 et 11,0 ; et/ou
- un ralentisseur de flux ; et/ou
- une base ; et/ou
- un sel; et/ou
- une solution de dilution appropriée, par exemple de l'eau.

Ainsi, la composition tampon peut comprendre ou consister en (i) un composé capable de complexer spécifiquement des résidus glucose d'hémoglobines glyquées et de leur apporter des charges négatives à pH alcalin, et (ii) un ou plusieurs et en particulier l'ensemble des composés choisis parmi : un composé tampon ayant un pKa compris entre 8,0 et 11,0, un ralentisseur de flux, une base, un sel (en particulier un sel de sodium, par exemple du chlorure de sodium), une solution de dilution appropriée (par exemple de l'eau) et leurs mélanges.

Le composé tampon est de préférence un composé zwittérionique. Il peut notamment être choisi parmi l'AMP, l'AMPD, l'AMPSO, la bicine, le CABS, le CAPS, le CAPSO, le CHES, l'HEPBS, la méthylamine, le TABS, le TAPS, la taurine, la tricine et le Tris; en particulier, on choisit le CAPS, le CAPSO ou le CHES, de préférence le CHES. Ces composés ont un fort pouvoir tampon aux pH cibles (pH 8-11) et sont particulièrement adaptés pour obtenir une bonne focalisation des hémoglobines.

La concentration du composé tampon dans la composition tampon est généralement comprise entre 20 et 500 mM, de préférence entre 50 et 400 mM, et plus préférablement entre 100 et 350 mM ou entre 150 et 300 mM, par exemple environ 200 mM ou environ 300 mM.

Le ralentisseur de flux est destiné à renforcer l'effet des différences de charge électrique afin d'obtenir une bonne résolution de séparation entre les différentes hémoglobines. Ce type de composé agit en diminuant le flux électro-osmotique, ce qui retarde la migration des différentes fractions et permet d'augmenter leur séparation. Le ralentisseur de flux utilisé peut être une diamine aliphatique, en particulier une diamine aliphatique choisie parmi les 1,3-diaminopropane, 1,4-diaminobutane (putrescine), 1,5-diaminopentane (cadavérine), 1-6-diaminohexane, la spermine et la DETA, ou encore un dérivé de cette diamine aliphatique ou un de leurs mélanges.

Par « dérivé », on entend ici notamment une polyamine aliphatique, une polyamine cyclique, un sel (par exemple un sel de sodium) ou un de leurs mélanges.

Selon un mode de réalisation particulier de l'invention, le ralentisseur de flux est choisi parmi la putrescine, ses dérivés et leurs mélanges.

Selon un mode de réalisation particulier de l'invention, le ralentisseur de flux est de la putrescine. En particulier, la putrescine est sous forme pure, avec, le cas échéant, adjonction d'un sel (en particulier un sel de sodium, par exemple du chlorure de sodium).

Selon un autre mode de réalisation particulier de l'invention, le ralentisseur de flux est de la putrescine chlorhydrate (ou putrescine 2HCl).

Dans la composition tampon de l'invention, la concentration du ralentisseur de flux est avantageusement comprise entre 0,10 et 40 mM, de préférence entre 10 et 30 mM et plus préférablement entre 15 et 25 mM, par exemple 20 mM.

La base éventuellement ajoutée dans la composition tampon permet d'ajuster le pH de ladite composition. On peut notamment utiliser une base appartenant à la famille des hydroxydes, en particulier une base choisie parmi l'hydroxyde de lithium, l'hydroxyde de sodium, l'hydroxyde de potassium, l'hydroxyde de rubidium, l'hydroxyde de césium, l'hydroxyde de francium, un hydroxyde de mono-, di-, tri- ou tétra-alkyl ammonium et leurs mélanges.

Selon un mode de réalisation particulier de l'invention, la base éventuellement ajoutée dans la composition tampon est de l'hydroxyde de sodium.

Avantageusement, on ajoute suffisamment de base dans la composition tampon pour que son pH soit supérieur ou égal à 9,0, de préférence compris entre 9,0 et 11,0 et plus préférablement compris entre 9,0 et 10,0, par exemple compris entre 9,3 et 9,5, et encore plus préférablement un pH de 9,4 ou 9,5. Un pH alcalin supérieur à 9 permet d'obtenir des fractions d'hémoglobines chargées négativement (le point isoélectrique des hémoglobines étant compris entre 6,05 et 7,63).

Selon un mode de réalisation particulier de l'invention, le sel éventuellement présent dans la composition tampon de l'invention est un sel de sodium, de préférence le chlorure de sodium. La composition tampon peut comporter par exemple 2.5 g/l de chlorure de sodium.

Selon un mode de réalisation particulier de l'invention, lorsque la composition tampon comporte de la putrescine, notamment de la putrescine pure, elle comporte également un sel (en particulier un sel de sodium), de préférence du chlorure de sodium, par exemple 2.5 g/l de chlorure de sodium.

Selon un autre mode de réalisation particulier de l'invention, la composition tampon comporte de la putrescine chlorhydrate, ne comporte pas de chlorure de sodium, et de préférence ne comporte aucun sel.

Selon un mode de réalisation particulier de l'invention, la composition tampon comprend ou consiste en :
- 200mM de CAPSO ;
- 10mM de putrescine (par exemple soit de la putrescine 2HCl, soit de la putrescine pure et du chlorure de sodium);
- 50mM d'acide 3,5-diCarboxyPhenylBoronique ;
- de l'eau ; et
- le cas échéant, une base, par exemple la soude, pour ajuster le pH, par exemple à une valeur supérieure à 9 ou 10, et de préférence à une valeur de 10.2 ;
ou, plus préférablement, en :
- 200mM de CAPSO ;
- 15mM de putrescine (par exemple soit de la putrescine 2HCl, soit de la putrescine pure et du chlorure de sodium);
- 100mM d'acide 3,5-diCarboxyPhenylBoronique ;
- de l'eau ; et
- le cas échéant, une base, par exemple la soude, pour ajuster le pH, par exemple à une valeur supérieure à 9 ou 10, et de préférence à une valeur de 10.2 ;
ou, encore plus préférablement, en :
- 200mM de CHES;
- 20mM de putrescine (par exemple soit de la putrescine 2HCl, soit de la putrescine pure et du chlorure de sodium);
- 30mM d'acide 3,5-diCarboxyPhenylBoronique ;
- de l'eau ; et
- le cas échéant, une base, par exemple la soude, pour ajuster le pH, par exemple à une valeur supérieure à 9, et de préférence à une valeur de 9.4.

Selon un mode de réalisation particulier de l'invention, la composition tampon comprend ou consiste en les constituants indiqués ci-dessus, dans les concentrations indiquées ci-dessus, et un ou plusieurs sels, en particulier du chlorure de sodium, par exemple 2.5 g/l de chlorure de sodium.

Comme illustré dans la partie exemple, de telles compositions tampon permettent d'obtenir une séparation parfaite de l'hémoglobine HbA_{1c} des autres hémoglobines présentes dans l'échantillon biologique analysé, ainsi qu'une séparation entre elles des différentes fractions mineures (dont l'HbA₁ₐ, l'HbA_{1b} et l'HbA_{1c}) inégalée dans le domaine de l'électrophorèse capillaire en solution libre.

Le terme « échantillon biologique » désigne, dans la présente demande, tout fluide biologique comportant des globules rouges. Ledit fluide biologique peut provenir d'un patient humain sain ou malade (par exemple un patient diabétique) ou être d'origine animale, en particulier provenir d'un mammifère non humain (sain ou malade). Ledit liquide biologique (humain ou de mammifère non humain) peut être par exemple du sang, en particulier du sang normal ou non, lavé, décanté, centrifugé, hémolysé ou total. Les échantillons biologiques peuvent aussi être d'origine synthétique ou purifiée.

L'invention est particulièrement utile pour l'analyse d'échantillons de sang, en particulier pour l'analyse d'un échantillon de sang provenant d'un patient diabétique ou d'un mammifère non humain diabétique.

Selon un mode de réalisation particulier, ledit échantillon biologique comprend ou consiste en plusieurs hémoglobines, en particulier plusieurs hémoglobines glyquées comportant un résidu glucose lié à l'acide aminé se trouvant en position N-terminale sur au moins une chaîne de globine (par exemple sur les chaînes bêta), et plus particulièrement de l'hémoglobine A_{1c}.

L'échantillon biologique utilisé peut être dilué préalablement à l'analyse par électrophorèse capillaire avec une solution de dilution appropriée, par exemple une solution hémolysante et/ou une solution de tampon d'analyse, en particulier la composition tampon employée dans le cadre de l'invention. On réalise de préférence l'analyse à partir d'un échantillon de sang hémolysé.

Selon un autre mode de réalisation particulier, l'échantillon biologique est dilué, dans un premier temps, dans une solution hémolysante, puis dans la composition tampon employée dans le cadre de l'invention.

Le terme « solution hémolysante » désigne, dans la présente demande, une solution capable de provoquer l'hémolyse des globules rouges, c'est à dire la destruction des globules rouges, et de libérer ainsi l'hémoglobine. Elle peut permettre, selon sa composition, une lyse totale des globules rouges, en usant éventuellement d'un faible mouvement mécanique additionnel (vortex, agitation,...). La solution hémolysante peut comprendre des additifs usuels de lyse cellulaire, comme par exemple le Triton X100, qui est communément utilisé à une concentration de 1g/L. La solution hémolysante peut en outre optionnellement comprendre un composé capable de complexer spécifiquement des résidus glucose d'hémoglobines glyquées et de leur apporter des charges négatives à pH alcalin tel que défini dans la présente demande.

A titre d'exemple, la solution hémolysante peut être choisie parmi le groupe constitué de la solution hémolysante Capillarys® Hemoglobin(e) de Sebia, la solution hémolysante MlNlCAP® Hemoglobin(e) de Sebia, la solution hémolysante Hydragel® HbA_{1c} de Sebia, l'eau pure, l'eau additionnée de tensioactif, en particulier l'eau additionnée de Triton X100 (par exemple 1g/L de triton X100) et leurs mélanges.

La méthode d'analyse par électrophorèse capillaire de l'invention comprend les étapes suivantes :
a) l'introduction, dans un capillaire d'électrophorèse, d'une composition tampon telle que définie en revendication 1 et de l'échantillon biologique ; et
b) la séparation des constituants dudit échantillon biologique par électrophorèse capillaire.

Ces deux étapes sont généralement précédées d'une étape de dilution de l'échantillon biologique, par exemple dans une solution hémolysante. Cette étape de dilution peut être réalisée notamment dans le support d'échantillon, par exemple, dans une barrette Capillarys® (Sebia) ou dans la cupule MlNlCAP® (Sebia).

A l'étape a), la composition tampon et l'échantillon biologique peuvent être introduits séparément (simultanément ou non) dans un même capillaire d'électrophorèse, le mélange étant alors réalisé dans le capillaire. On peut, par exemple, introduire, dans le capillaire d'électrophorèse, d'abord la composition tampon employée dans le cadre de l'invention, puis l'échantillon.

Alternativement, l'échantillon biologique peut être introduit dans un capillaire d'électrophorèse à l'étape a) sous la forme d'un mélange avec la composition tampon.

Selon le type d'appareil d'électrophorèse capillaire utilisé et en fonction du nombre d'analyses à réaliser, on utilise un seul capillaire ou plusieurs capillaires en parallèle. Lorsqu'on dispose d'un seul capillaire, ce capillaire est généralement utilisé plusieurs fois successivement, de façon à pouvoir réaliser plusieurs analyses. Bien entendu, lorsqu'on utilise plusieurs capillaires, on peut le cas échéant réaliser plusieurs migrations électrophorétiques successives, au cours desquelles plusieurs capillaires sont utilisés en parallèle.

L'étape de séparation des constituants de l'échantillon biologique par électrophorèse capillaire consiste notamment à appliquer au(x) capillaire(s) un champ électrique de voltage suffisant pour permettre la séparation d'une ou plusieurs hémoglobine(s) glyquée(s) d'intérêt des autres protéines et en particulier des autres hémoglobines qui peuvent être présentes dans l'échantillon biologique.

Les conditions de réalisation de l'électrophorèse capillaire en veine liquide sont les conditions habituellement mises en œuvre par l'homme du métier pour les étapes d'introduction de l'échantillon et de la composition tampon dans le ou les capillaire(s) et de séparation des constituants de l'échantillon par migration électrophorétique Le champ électrique appliqué peut être par exemple d'environ 400 V/cm. Elles peuvent comporter usuellement un lavage des capillaires par une solution de lavage, un lavage avec la composition tampon utilisée pour l'analyse, une ou des dilution(s) éventuelle(s) de l'échantillon, l'introduction de l'échantillon dans le ou les capillaire(s), la migration et la détection. Ces étapes peuvent être réalisées par des automates.

Des conditions de réalisation d'une éléctrophorèse capillaire sont par exemple les conditions appropriées pour utiliser l'automate Capillarys® de Sebia ou MlNlCAP® de Sebia.

Selon l'invention, l'étape de séparation des constituants de l'échantillon biologique est suivie d'une étape de détection d'une ou plusieurs protéine(s), en particulier d'une ou plusieurs hémoglobines présente(s) dans l'échantillon biologique, et plus particulièrement d'une ou plusieurs hémoglobine(s) glyquée(s) d'intérêt présente(s) dans l'échantillon biologique, par exemple l'hémoglobine A1c.

Cette étape de détection peut se faire notamment en mesurant l'absorbance, par exemple à une longueur d'onde d'environ 415 nm, spécifique de l'hémoglobine ; les hémoglobines peuvent être analysées à une longueur d'onde d'environ 200 nm, mais pour éviter des interférences avec notamment les protéines plasmatiques, elles sont de préférence analysées à une longueur d'onde de 415 nm.

Dans le cadre de la réalisation de l'invention, la présentation des résultats de la séparation électrophorétique peut être faite comme illustrée dans les exemples, sous la forme de profils électrophorétiques générés à partir d'un signal de détection proportionnel à la quantité d'hémoglobine détectée. Ainsi, la méthode de l'invention comprend une étape de génération d'un électrophorégramme à partir du signal de détection.

Comme indiqué précédemment, lorsqu'elle est présente dans l'échantillon biologique analysé, une hémoglobine d'intérêt peut être quantifiée. Pour ce faire, on détermine la surface du pic correspondant à ladite hémoglobine.

Ainsi, ladite méthode comprend généralement également une étape de détermination, en particulier à partir de l'électrophorégramme, de la quantité d'une ou plusieurs hémoglobine(s) présente(s) dans l'échantillon biologique et/ou de la proportion d'une ou plusieurs hémoglobine(s) présente(s) dans l'échantillon biologique par rapport à la quantité totale de protéines, à la quantité totale d'hémoglobine et/ou à la quantité de certaines d'hémoglobines (par exemple par rapport à la quantité d'HbA ou d'HbA₀) présentes dans l'échantillon biologique. On peut notamment déterminer la proportion d'hémoglobine glyquée A_{1c}, S_{1c} et/ou C_{1c} présente dans l'échantillon biologique par rapport à la quantité totale de toutes les hémoglobines ou par rapport à la quantité de certaines hémoglobine(s) présente(s) dans l'échantillon biologique, par exemple par rapport à la quantité totale d'hémoglobine A, S et/ou C respectivement.

Dans un mode de réalisation particulier de l'invention, ce dosage peut être obtenu directement à partir du profil électrophorétique.

Selon un mode de réalisation particulier de l'invention, la méthode d'analyse comprend en outre une étape de quantification d'une ou plusieurs hémoglobine(s) présente(s) dans l'échantillon biologique analysé par rapport à un ou plusieurs calibrateur(s) standardisé(s) (par exemple un ou plusieurs échantillons biologiques de référence); ceci permet de standardiser les résultats.

Ainsi, afin d'obtenir un niveau de précision élevé lors du dosage, chaque capillaire est généralement calibré à chaque démarrage de l'appareil d'électrophorèse en utilisant des échantillons biologiques de référence (par exemple des échantillons standards contenant des concentrations connues de différentes fractions d'hémoglobine glyquée naturelles et/ou synthétiques, et le cas échéant purifiées). Par exemple, pour doser l'HbA_{1c}, on utilise généralement au moins deux calibrateurs, par exemple un premier calibrateur comprenant une forte concentration connue d'HbA_{1c}, et un deuxième calibrateur comprenant une faible concentration connue d'HbA_{1c}. On mesure alors, avec chaque capillaire de l'appareil d'électrophorèse, la quantité d'HbA_{1c} présente dans chaque calibrateur. Cela permet d'établir, pour chaque capillaire, une courbe de régression (sur au moins deux points), qui permettra ensuite de normaliser les mesures effectuées en utilisant ces différents capillaires. Ceci permet également de standardiser les résultats obtenus par rapport à une méthode de référence, en utilisant les valeurs d'HbA1c déterminées par cette méthode pour les calibrateurs.

Selon un mode de réalisation particulier de l'invention, l'électrophorèse capillaire réalisée est du type électrophorèse capillaire en solution libre, en particulier du type électrophorèse capillaire en solution libre en capillaire(s) nu(s).

Du point de vue des matériaux utilisés pour les capillaires, ceux-ci sont usuels en électrophorèse capillaire. Le spécialiste saura adapter la nature du capillaire et sa taille aux besoins de l'analyse.

Par exemple, dans un mode de réalisation particulier, le ou les capillaire(s) d'électrophorèse est (sont) en silice fondue.

Est également décrit l'utilisation de la méthode d'analyse par électrophorèse capillaire selon la présente demande pour séparer une ou plusieurs hémoglobine(s) glyquée(s) comportant du glucose et plus précisément une ou plusieurs hémoglobine(s) glyquée(s) comportant chacune, sur une ou plusieurs chaîne(s) de globine (par exemple sur les chaînes bêta), un ou plusieurs résidu(s) glucose lié(s) à l'acide aminé se trouvant en position N-terminale, d'autres protéines, en particulier d'au moins une autre hémoglobine et de préférence des autres hémoglobines présente(s) dans un échantillon biologique et, le cas échéant, pour doser ladite ou lesdites hémoglobine(s) glyquée(s).

La présente invention a également pour objet une composition tampon appropriée pour l'analyse, par électrophorèse capillaire, d'un échantillon biologique comprenant des hémoglobines et en particulier une ou plusieurs hémoglobine(s) glyquée(s) comportant un ou plusieurs glucose sur une ou sur plusieurs chaînes de globine bêta, la composition tampon étant telle que définie en revendication 12 ou en revendication 13.

Ladite composition tampon est notamment celle utilisée pour mettre en oeuvre la méthode d'analyse par électrophorèse capillaire d'un échantillon biologique présentée dans la présente demande.

Selon un mode de réalisation particulier de l'invention, la composition tampon comprend, à titre de composé capable de complexer spécifiquement des résidus glucose et d'apporter des charges électriques négatives à pH alcalin, un dérivé boronique d'acide polycarboxylique, en particulier un acide dicarboxylique ou un acide tricarboxylique, par exemple l'acide 3,5-diCarboxyPhenylBoronique.

Selon un autre mode de réalisation particulier de l'invention, la composition tampon comprend, outre le composé capable de complexer spécifiquement des résidus glucose et d'apporter des charges électriques négatives à pH alcalin,
- un ralentisseur de flux ; et/ou
- un composé tampon ayant pKa compris entre 8,0 et 11,0 ; et/ou
- une base ; et/ou
- un sel (en particulier un sel de sodium, par exemple du chlorure de sodium) ; et/ou
- une solution de dilution appropriée, par exemple de l'eau.

Ces différents constituants peuvent être tels que définis dans l'une des revendications 4 à 8.

Selon un mode de réalisation particulier de l'invention, la composition tampon a un pH supérieur ou égal à 9,0, de préférence un pH compris entre 9,0 et 11,0 et plus préférablement un pH compris entre 9,0 et 10,0, par exemple un pH compris entre 9,3 et 9,5, et encore plus préférablement un pH de 9,4. Un tel pH peut être notamment obtenu par l'apport d'une quantité suffisante d'une base telle que définie ci-dessus.

Les compositions tampon de l'invention sont préparées de façon usuelle pour des compositions de tampon d'analyse, à savoir par adjonction des constituants sous forme liquide, ou solide à diluer, à un support acceptable. De façon usuelle, le support est de l'eau, distillée ou déminéralisée.

La présente invention concerne en outre une trousse (ou kit) tel(le) que défini(e) en revendication 14, comprenant une composition tampon de l'invention et, le cas échéant, une notice d'utilisation, pour réaliser l'analyse électrophorétique. En d'autres termes, la trousse de l'invention peut comprendre ou consister en une composition tampon telle que revendiquée et un matériau d'emballage et, le cas échéant, une notice d'utilisation.

La trousse de l'invention peut en outre comprendre une ou plusieurs solution(s) de lavage des capillaires et/ou une ou plusieurs barrette(s) de dilution et/ou une ou plusieurs solution(s) appropriée(s) pour diluer l'échantillon biologique à analyser (par exemple une solution hémolysante, en particulier une solution hémolysante telle que définie dans la présente demande) et/ou une ou plusieurs échantillon(s) biologique(s) de référence (par exemple des fractions glyquées naturelles et/ou synthétiques, et le cas échéant purifiées) permettant de calibrer chaque capillaire.

La présente demande décrit également l'utilisation d'une composition tampon telle que définie dans la présente demande et/ou d'une trousse telle que définie dans la présente demande, pour l'analyse, par électrophorèse capillaire, d'hémoglobines glyquées (une ou plusieurs) comportant un ou plusieurs résidu(s) glucose, en particulier des hémoglobines A_{1c}, S_{1c} et C_{1c}, contenues dans un échantillon biologique comprenant une ou plusieurs hémoglobine(s).

La présente invention a également pour objet, comme défini en revendication 15, l'utilisation d'une composition tampon telle que définie dans les revendications et/ou d'une trousse telle que définie dans les revendications, pour séparer, par électrophorèse capillaire, une ou plusieurs hémoglobine(s) glyquée(s) comportant au moins une chaîne de globine bêta comportant un résidu glucose lié à l'acide aminé se trouvant en position N-terminale, et plus particulièrement pour séparer, par électrophorèse capillaire, l'hémoglobine A_{1c}, d'autres protéines, en particulier d'au moins une autre hémoglobine et de préférence des autres hémoglobines présente(s) dans un échantillon biologique, et, le cas échéant, pour doser la ou lesdites hémoglobine(s) ainsi séparée(s).

La composition tampon telle que définie dans les revendications, la trousse telle que définie dans les revendications sont également appropriés pour une utilisation, par exemple dans le cadre d'une méthode de l'invention, pour le diagnostique du diabète chez un humain ou un mammifère non humain et/ou pour le suivi de l'équilibre glycémique d'un humain ou d'un mammifère non humain (en particulier un sujet diabétique), comme défini en revendication 16, notamment pour évaluer l'efficacité d'un traitement contre le diabète et/ou adapter un traitement contre le diabète chez un sujet diabétique. Le ou les échantillon(s) biologique(s) analysés proviennent alors dudit humain ou dudit mammifère non humain.

Le terme diabète tel qu'utilisé dans la présente demande désigne un diabète de type 1 et/ou un diabète de type 2.

La présente demande décrit également l'utilisation d'une composition tampon, d'une trousse et/ou d'un composé capable de complexer spécifiquement des résidus glucose d'hémoglobines glyquées et d'apporter des charges négatives à pH alcalin, pour la fabrication d'un kit de diagnostique. Ledit kit de diagnostique peut notamment être utilisé pour le diagnostique du diabète chez un humain ou d'un mammifère non humain et/ou pour contrôler l'équilibre glycémique d'un humain ou d'un mammifère non humain, en particulier un sujet diabétique. Ledit kit peut ainsi permettre d'évaluer l'efficacité d'un traitement contre le diabète chez un humain ou un mammifère non humain atteint de diabète et/ou d'adapter un traitement contre le diabète chez un sujet diabétique.

Le taux d'HbA_{1c} est généralement compris entre 4 et 6% (soit 20 à 42 mmoles d'HbA_{1c} par moles d'hémoglobine dans le sang) chez un humain non diabétique, et supérieur à 7% chez un patient diabétique (en l'absence de traitement).

En cas de taux d'HbA1c compris entre 6 et 7% (soit une concentration de 42 à 53 mmoles d'HbA1c par moles d'hémoglobine dans le sang), il est conseillé de mettre en place un traitement anti-diabétique.

Au-delà du seuil de 8%, qui équivaut à une concentration de 64 mmoles d'HbA_{1c} par moles d'hémoglobine dans le sang (Panteghini et John, 2007), le patient s'expose à un risque accru de développer une des complications du diabète (microangiopathie, macroangiopathie...). Il est alors conseillé de modifier le traitement anti-diabétique du patient.

D'autres caractéristiques et avantages de l'invention apparaissent dans les exemples qui suivent ainsi que dans les figures qui illustrent la réalisation de l'invention.

### DESCRIPTION DES FIGURES

Figure 1 : électrophérogramme obtenu sur CE Agilent à partir d'un échantillon de sang humain quelconque en utilisant le tampon d'analyse décrit dans le brevet US 5,599,433, qui contient 100mM de CAPS et 300mM d'acide borique (pH : 11.00 ; température : 24°C ; voltage : 6.1 kV, soit 190V/cm ; injection : 50 mbars 20s).
Figure 2 : profils électrophorétiques standards A_{1a,b} et A_{1c} obtenus sur CE Agilent à partir d'échantillons de référence contenant différentes fractions d'hémoglobine glyquée purifiées en utilisant le tampon d'analyse décrit dans le brevet US 5,599,433, qui contient 100mM de CAPS et 300mM d'acide borique (pH : 11 ; température : 24°C ; voltage : 6.1 kV, soit 190V/cm ; injection : 50 mbars 20s).

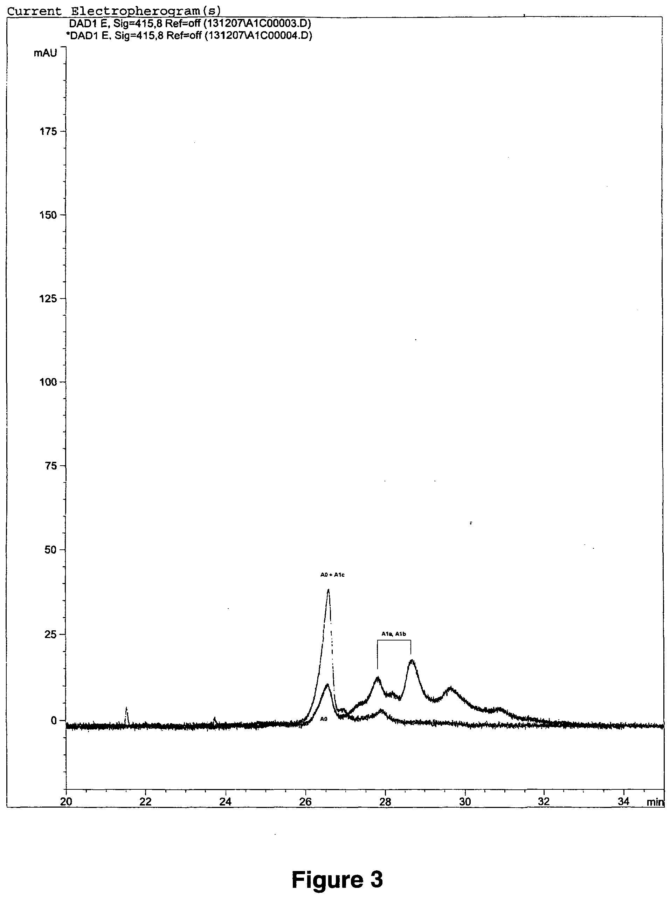

Figure 3 : profils électrophorétiques standards A_{1a,b} et A_{1c} obtenus sur CE Agilent à partir d'échantillons de référence contenant différentes fractions d'hémoglobine glyquée purifiées, en utilisant une composition tampon contenant 200mM de CAPSO et 10mM de putrescine mais pas de composé borate, ni de composé boronate (pH : 10.20 ; température : 24°C ; voltage : 6.1 kV, soit 190V/cm ; injection : 50 mbars 20s).

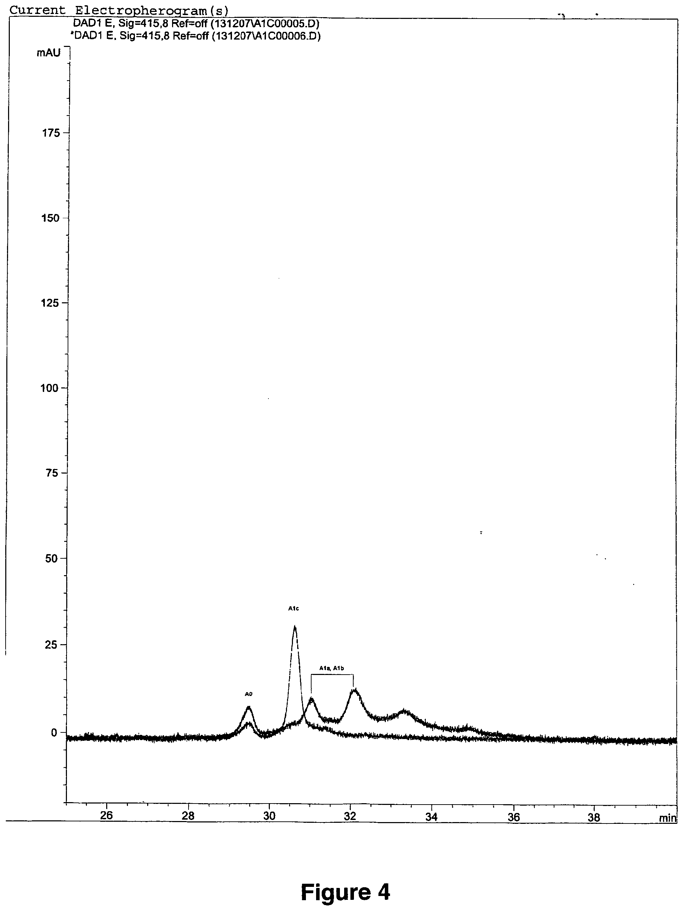

Figure 4 : profils électrophorétiques standards A_{1a,b} et A_{1c} obtenus sur CE Agilent à partir d'échantillons de référence contenant différentes fractions d'hémoglobine glyquée purifiées, en utilisant une composition tampon contenant 200mM de CAPSO, 10mM de putrescine et 50mM de borate (pH : 10.20 ; température : 24°C ; voltage : 6.1 kV, soit 190V/cm ; injection : 50 mbars 20s).

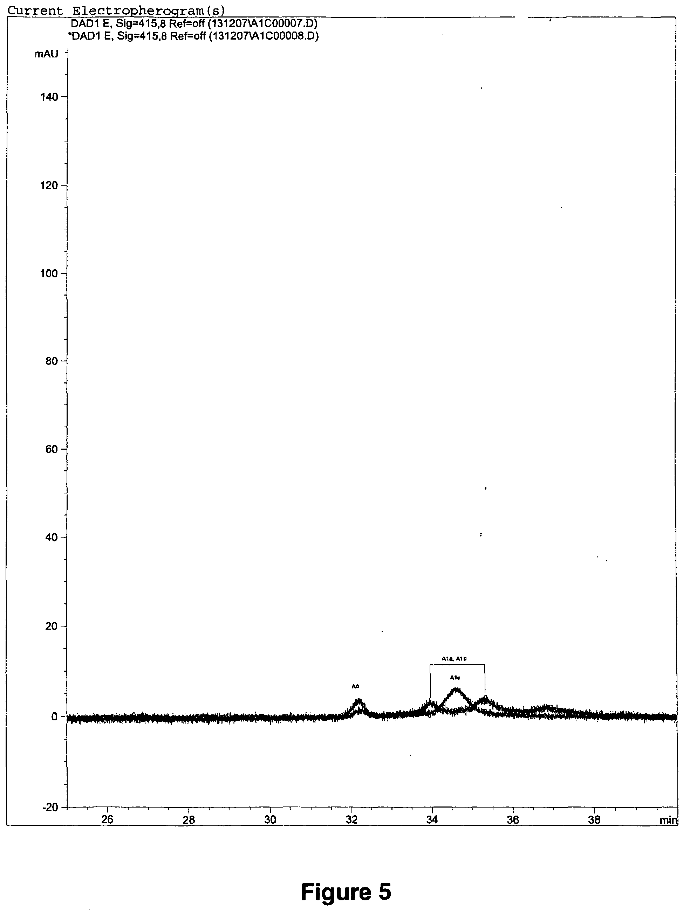

Figure 5 : profils électrophorétiques standards A_{1a,b} et A_{1c} obtenus sur CE Agilent à partir d'échantillons de référence contenant différentes fractions d'hémoglobine glyquée purifiées, en utilisant une composition tampon contenant 200mM de CAPSO, 10mM de putrescine et 50mM d'acide 3-CarboxyPhenylBoronique (pH : 10.20 ; température : 24°C ; voltage : 6.1 kV, soit 190V/cm ; injection : 50 mbars 20s).

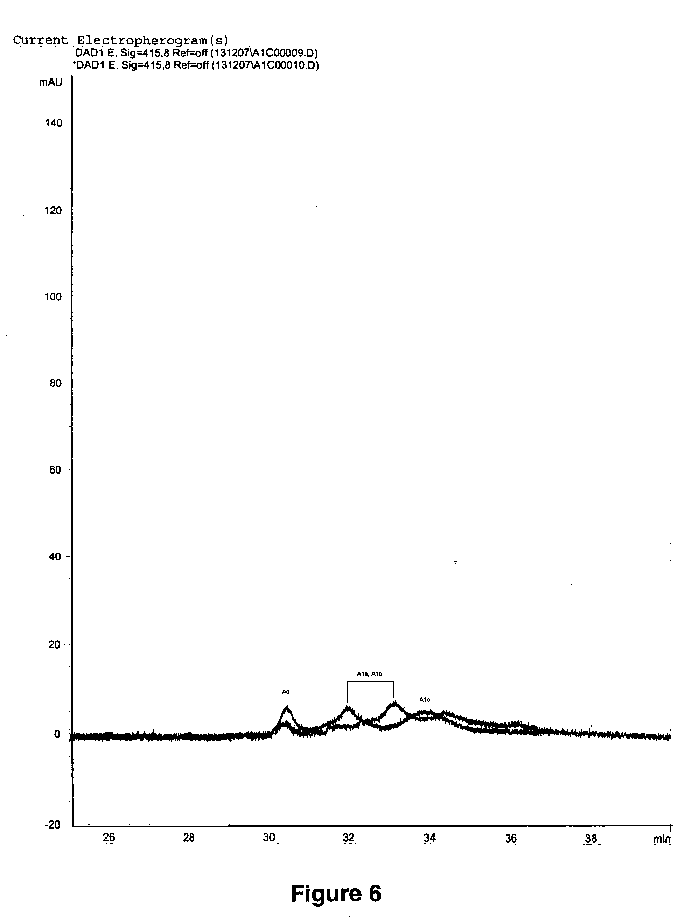

Figure 6 : profils électrophorétiques standards A_{1a,b} et A_{1c} obtenus sur CE Agilent à partir d'échantillons de référence contenant différentes fractions d'hémoglobine glyquée purifiées, en utilisant une composition tampon contenant 200mM de CAPSO, 10mM de putrescine et 50mM d'acide 3,5-diCarboxyPhenylBoronique (pH : 10.20 ; température : 24°C ; voltage : 6.1 kV, soit 190V/cm ; injection : 50 mbars 20s).

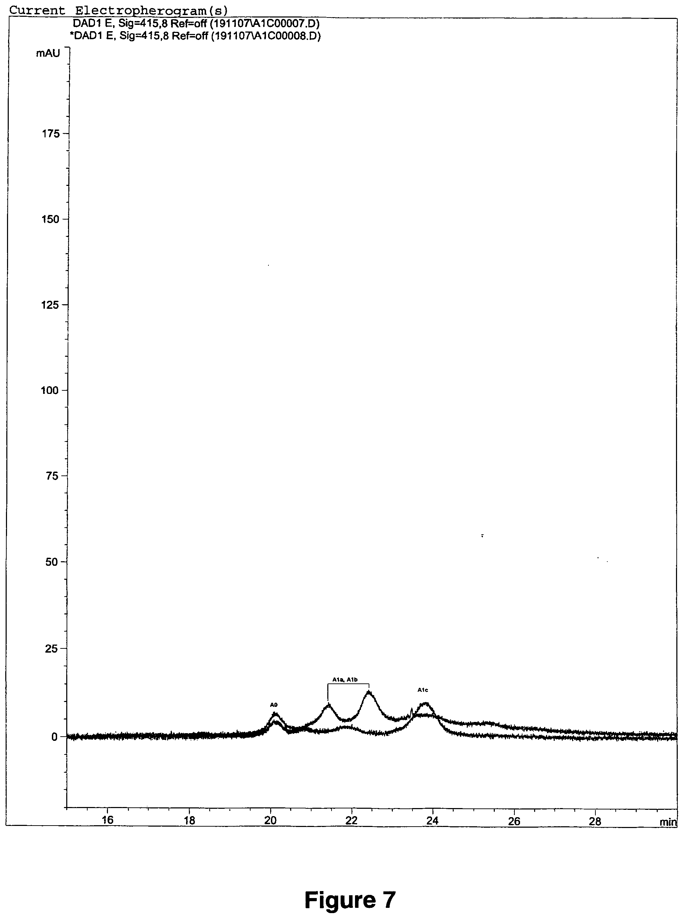

Figure 7 : profils électrophorétiques standards A_{1a,b} et A_{1c} obtenus sur CE Agilent à partir d'échantillons de référence contenant différentes fractions d'hémoglobine glyquée purifiées, en utilisant une composition tampon contenant 200mM de CAPSO, 15mM de putrescine et 100mM d'acide 3,5-diCarboxyPhenylBoronique (pH : 10.20 ; température : 30°C ; voltage : 10 kV, soit 310V/cm ; injection : 50 mbars 20s).

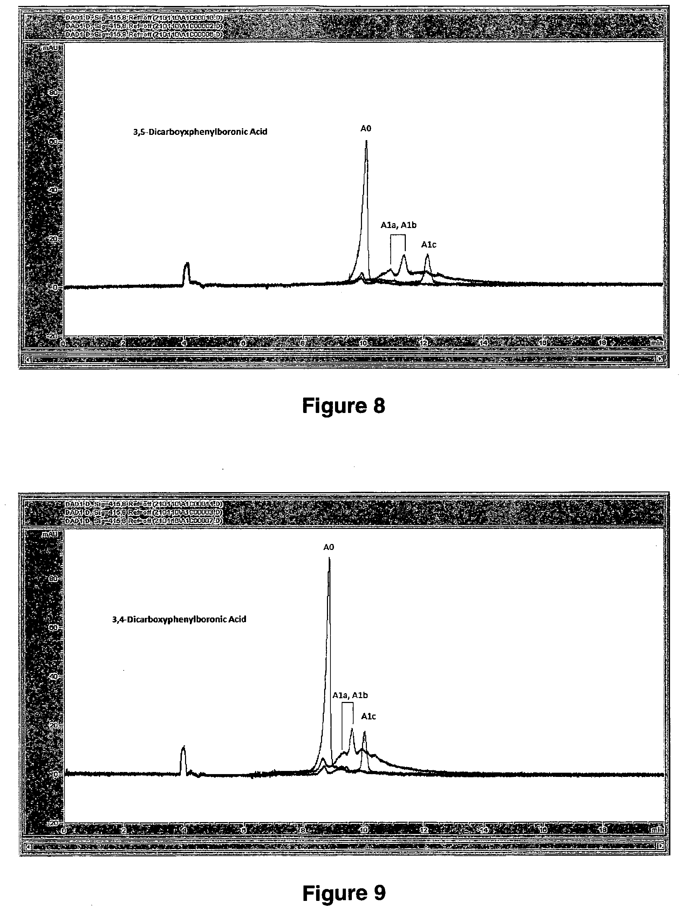

Figure 8 : profils électrophorétiques standards A₀, A_{1a,b} et A_{1c} obtenus sur CE Agilent à partir d'échantillons de référence contenant de l'HbAo et/ou différentes fractions d'hémoglobine glyquée purifiées, en utilisant une composition tampon contenant 200mM de CHES, 20mM de putrescine et 30mM d'acide 3,5-diCarboxyPhenylBoronique à pH : 9.40. Capillaire en silice fondue, non coaté (température: 34°C ; voltage: 17.3 kV soit 520V/cm ; injection 50mbars 20s).
Figure 9 : profils électrophorétiques standards A₀, A_{1a,b} et A_{1c} obtenus sur CE Agilent à partir d'échantillons de référence contenant de l'HbAo et/ou différentes fractions d'hémoglobine glyquée purifiées, en utilisant une composition tampon contenant 200mM de CHES, 20mM de putrescine et 30mM d'acide 3,4-diCarboxyPhenylBoronique à pH : 9.40. Capillaire en silice fondue, non coaté (température: 34°C ; voltage: 17.3 kV soit 520V/cm ; injection 50mbars 20s).

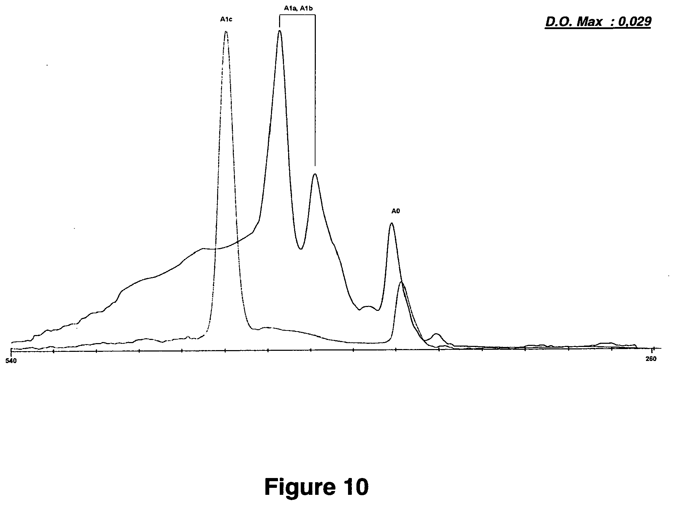

Figure 10 : profils électrophorétiques standards A_{1a,b} et A_{1c} obtenus sur Capillarys® (Sebia) à partir d'échantillons de référence contenant différentes fractions d'hémoglobine glyquée purifiées, en utilisant une composition tampon contenant 200mM de tampon CHES, 20mM de putrescine et 30mM d'acide 3,5-diCarboxyPhenylBoronique, à pH 9.40. Capillaire en silice fondue, non coaté (température : 34°C ; voltage : 9.4kV, soit 520 V/cm ; injection 20 mbars 6s).

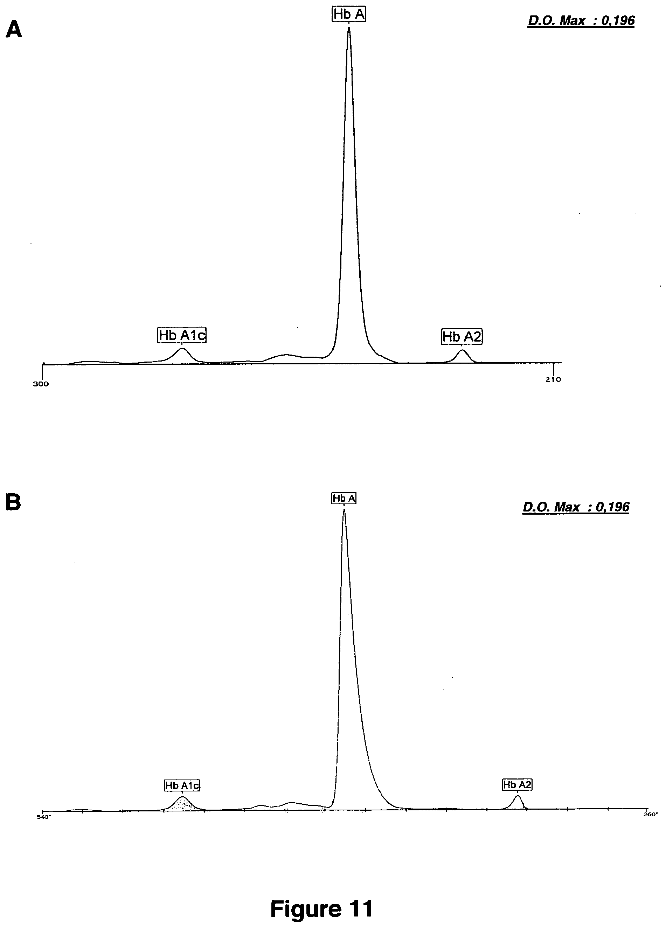

Figure 11 : électrophérogramme obtenu sur Capillarys® (Sebia) à partir d'un échantillon de sang humain quelconque dilué au 1/6^{ième} dans la solution hémolysante (eau + 1g/L de Triton X100), en utilisant une composition tampon contenant (A) 200 mM de tampon CAPSO, 10 mM de putrescine et 50 mM d'acide 3,5-diCarboxyPhenylBoronique à pH 10.2, ou, (B) 200mM de tampon CHES, 20mM de putrescine et 30mM d'acide 3,5-diCarboxyPhenylBoronique, à pH 9.40. Capillaire en silice fondue, non coaté (température : 34°C ; voltage : 9.4kV, soit 520 V/cm ; injection 8 mbars 6s).

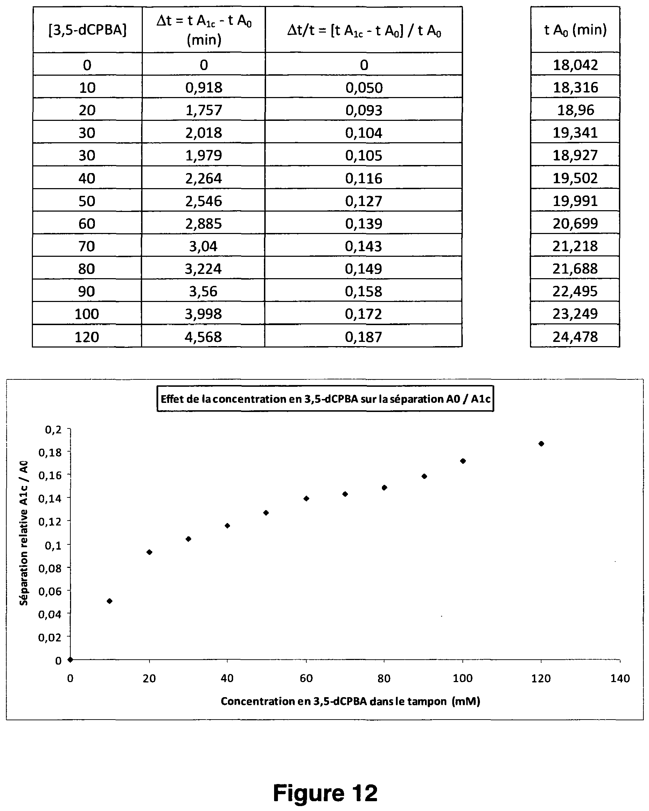

Figure 12 : étude de l'influence de la concentration en acide 3,5-diCarboxyPhenylBoronique dans la composition tampon sur la séparation entre les hémoglobines A_{1c} et Aₒ (température : 30 °C ; voltage : 10kV, soit 310V/cm).

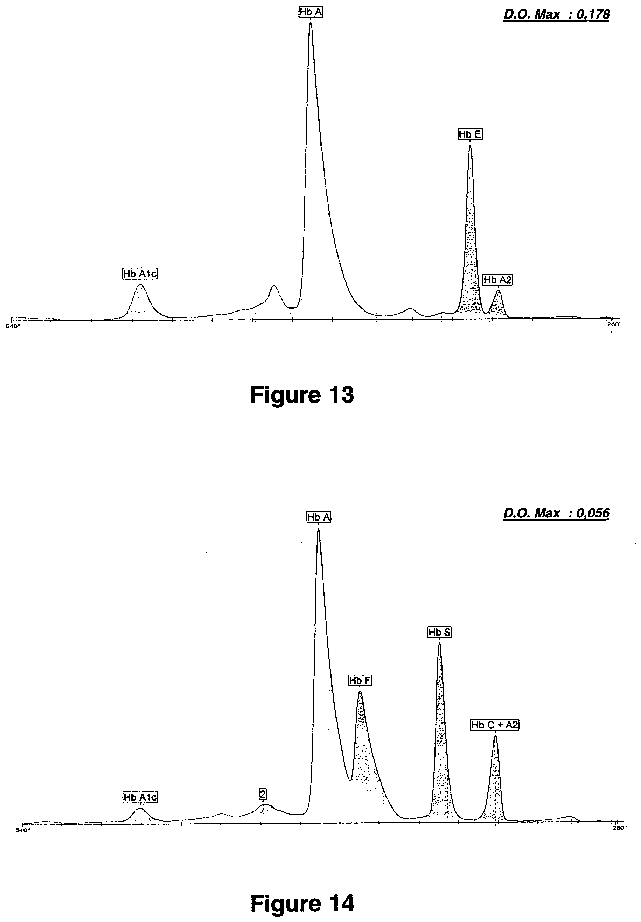

Figure 13 : électrophérogramme obtenu sur Capillarys® (Sebia) à partir d'un échantillon de sang humain quelconque comprenant un variant HbE, dilué au 1/6^{ième} dans la solution hémolysante (eau + 1g/L de Triton X100), en utilisant une composition tampon contenant 200mM de tampon CHES, 20mM de putrescine et 30mM d'acide 3,5-diCarboxyPhenylBoronique, à pH 9.40, et un capillaire en silice fondue, non coaté (température : 34°C ; voltage : 9.4kV, soit 520 V/cm ; injection 8 mbars 6s).
Figure 14 : électrophérogramme obtenu sur Capillarys® (Sebia) à partir d'un contrôle AFSC dilué au 1/6^{ième} dans la solution hémolysante (eau + 1g/L de Triton X100), en utilisant une composition tampon contenant 200mM de tampon CHES, 20mM de putrescine et 30mM d'acide 3,5-diCarboxyPhenylBoronique, à pH 9.40, et un capillaire en silice fondue, non coaté (température : 34°C ; voltage : 9.4kV, soit 520 V/cm ; injection 8 mbars 6s).

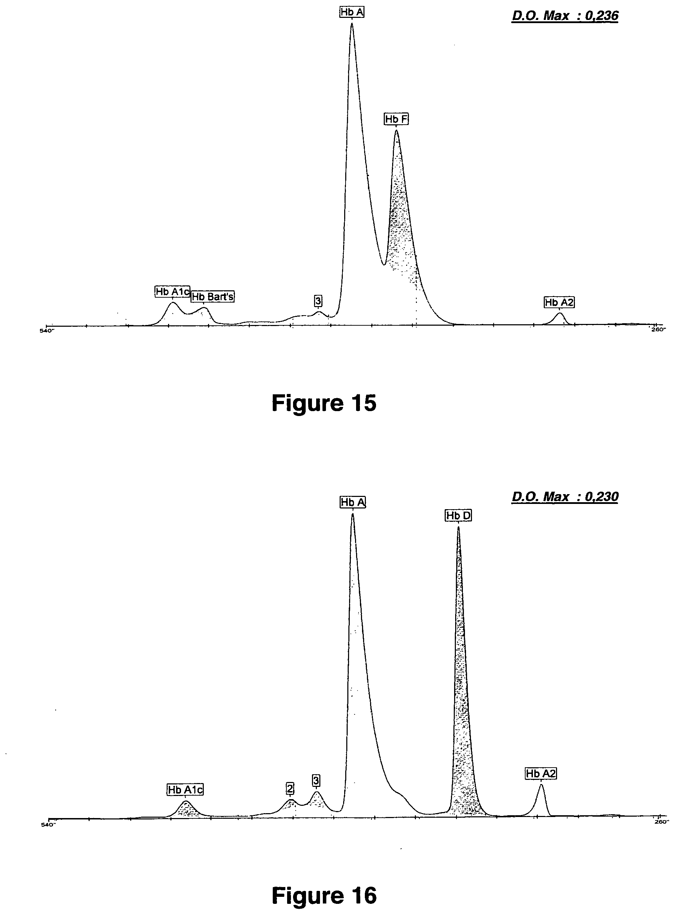

Figure 15 : électrophérogramme obtenu sur Capillarys® (Sebia) à partir d'un pool de sangs quelconques comportant des variants F et Bart's, dilué au 1/6^{ième} dans la solution hémolysante (eau + 1g/L de Triton X100), en utilisant une composition tampon contenant 200mM de tampon CHES, 20mM de putrescine et 30mM d'acide 3,5-diCarboxyPhenylBoronique, à pH 9.40, et un capillaire en silice fondue, non coaté (température : 34°C ; voltage : 9.4kV, soit 520 V/cm ; injection 8 mbars 6s).
Figure 16 : électrophérogramme obtenu sur Capillarys® (Sebia) à partir d'un échantillon de sang humain quelconque comprenant un variant HbD, dilué au 1/6^{ième} dans la solution hémolysante (eau + 1g/L de Triton X100), en utilisant une composition tampon contenant 200mM de tampon CHES, 20mM de putrescine et 30mM d'acide 3,5-diCarboxyPhenylBoronique, à pH 9.40, et un capillaire en silice fondue, non coaté (température : 34°C ; voltage : 9.4kV, soit 520 V/cm ; injection 8 mbars 6s).

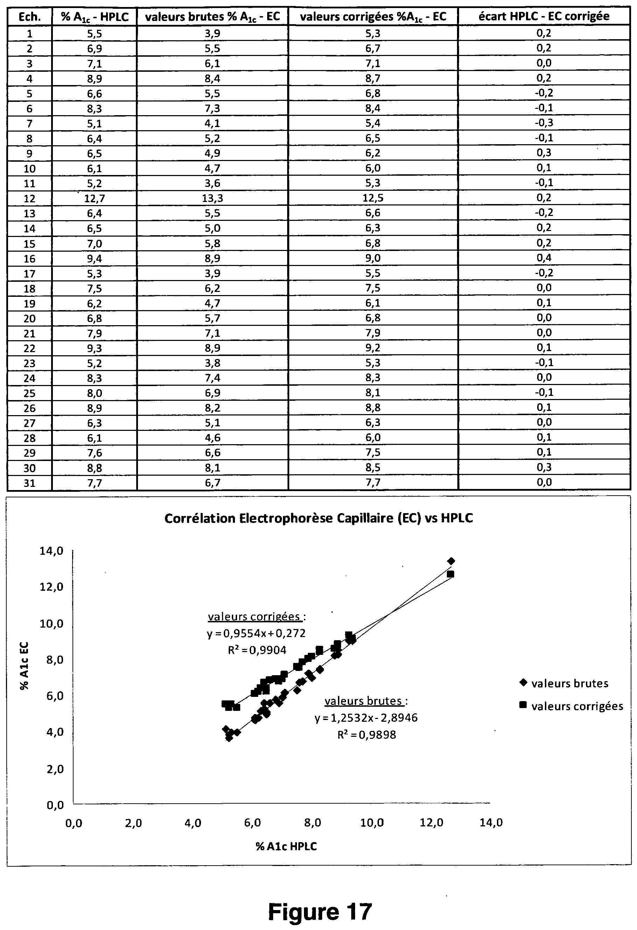

Figure 17 : comparaison des résultats obtenus par les inventeurs par électrophorèse capillaire avec l'analyseur Capillarys® (Sebia) aux résultats obtenus par HPLC avec l'analyseur Variant II Turbo® (Bio-Rad).

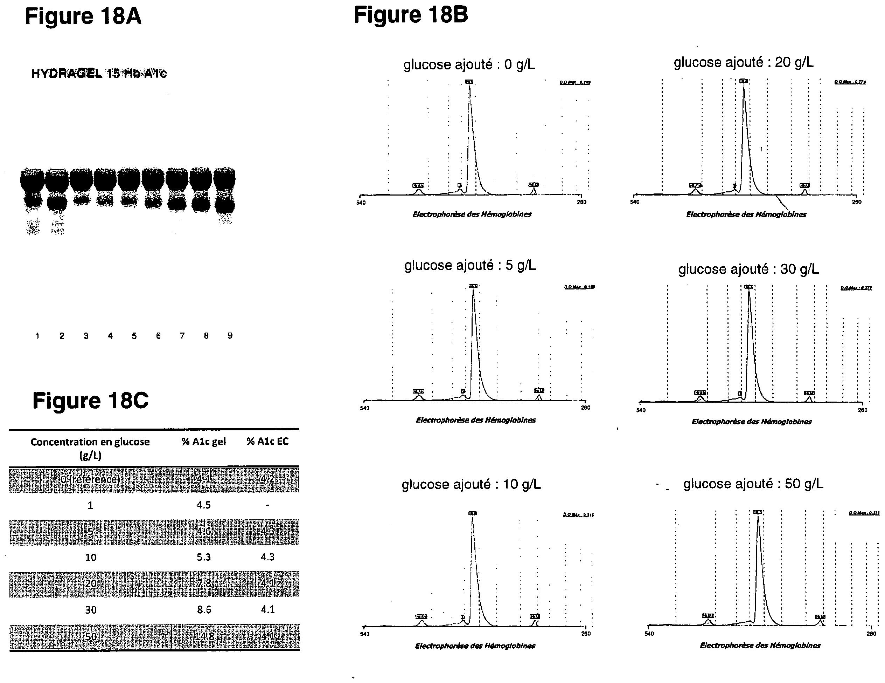

Figure 18A: Gel d'agarose HbA_{1c} réalisé sur automate Hydrasys® (Sebia). Pistes 1 et 2 : calibrateur A_{1c} faible (5.0%) et calibrateur A_{1c} fort (10.8%). Pistes 3 à 9 : sang quelconque total incubé 3h à 37°C avec du glucose à une concentration de 0g/L (référence ; piste 3), 1g/L (piste 4), 5g/L (piste 5), 10g/L (piste 6), 20g/L (piste 7), 30g/L (piste 8) et 50g/L (piste 9).
Figure 18B : profils d'électrophorèse capillaire obtenus avec l'analyseur Capillarys® (Sebia) avec les mêmes échantillons que ceux de la figure 18A. L'analyse n'a toutefois pas été réalisée pour l'échantillon contenant 1g/L de glucose car il n'y pas de différence visible en gel (figure 18A). Echantillons dilués au 1/6^{ième} dans la solution hémolysante (eau + 1g/L de Triton X100), en utilisant une composition tampon contenant 200mM de tampon CHES, 20mM de putrescine et 30mM d'acide 3,5-diCarboxyPhenylBoronique, à pH 9.40, et un capillaire en silice fondue, non coaté (température : 34°C ; voltage : 9.4kV, soit 520 V/cm ; injection 8 mbars 6s).
Figure 18C : tableau récapitulatif des valeurs d'HbA1c obtenues sur gel d'agarose et avec l'analyseur Capillarys® (Sebia) pour les analyses des sangs présentées dans les figures 18A et 18B.

### EXEMPLES

### A. MATERIEL ET METHODES

### ELECTROPHORESE CAPILLAIRE

Le principe de séparation est l'électrophorèse capillaire en solution libre à pH alcalin (pH > 9), afin d'obtenir des fractions d'hémoglobines chargées négativement (le point isoélectrique des hémoglobines est compris entre 6,05 et 7,63).

L'électrophorèse capillaire d'échantillons biologiques est réalisée sur un appareil d'électrophorèse capillaire équipé de 8 capillaires en silice fondue de diamètre interne 25 microns, de longueur utile 16 cm et de longueur totale 18 cm (système d'électrophorèse capillaire Capillarys® (Sebia) ou sur un appareil d'électrophorèse capillaire équipé d'un capillaire en silice fondue de diamètre interne 25 microns, de longueur utile 24 cm et de longueur totale 32 cm (système d'électrophorèse capillaire ^{3D}CE d'Agilent Technologies).

La détection est réalisée à une longueur d'onde de 415nm. Les échantillons de sang sont dilués dans une solution hémolysante (Triton X100 1 g/L dans l'eau) et injectés par injection hydrodynamique. Le capillaire est lavé avant chaque analyse par la soude 0,25 M, puis par la composition tampon.

### COMPOSITION TAMPON

Les compositions tampon dans lesquelles l'électrophorèse capillaire est réalisée comprend de l'eau, un composé tampon de pKa compris entre 8 et 11 (CAPS, CAPSO ou CHES selon les cas), une base permettant d'ajuster le pH à la valeur souhaitée, éventuellement un ralentisseur de flux (putrescine), et éventuellement un composé borate (acide borique) ou boronate.

L'acide 3,5-diCarboxyPhenylBoronique (3,5-dCPBA) a été obtenu auprès des sociétés Combi-blocks Inc. (San Diego, USA) et Apollo Scientific Ltd (Cheshire, Royaume-Uni).

L'acide 3,5-diCarboxyPhenylBoronique (3,4-dCPBA) a été synthétisé par la société BoroChem SAS (Caen, France).

### B. RESULTATS

### Exemple 1

Une électrophorèse capillaire a été réalisée à partir d'un sang humain quelconque (comportant des hémoglobines HbA₀, HbA₁ et HbA₂) dilué au 1/6^{ième} dans une solution hémolysante (1g/L de Triton X100 dissout dans de l'eau déminéralisée), en utilisant le tampon d'analyse décrit dans le brevet US 5,599,433, qui contient 100mM de CAPS et 300mM d'acide borique, pH 11. L'électrophérogramme obtenu est présenté en figure 1. On constate que la séparation entre les pics d'hémoglobines est mauvaise.

### Exemple 2

Une électrophorèse capillaire a été réalisée à partir d'échantillons de référence (Exocell, USA), contenant différentes fractions d'hémoglobine glyquée purifiées (fractions A₀ et A_{1c} ou fractions A₀, A_{1b} et A₁ₐ), en utilisant le tampon d'analyse décrit dans le brevet US 5,599,433, qui contient 100mM de CAPS et 300mM d'acide borique, pH 10.20. Les profils électrophorétiques standards A_{1a,b} et A_{1c} obtenus sont présentés en figure 2. Il apparaît clairement que la séparation entre les hémoglobines HbA_{1c} et HbA_{1a,b} est insuffisante ; le pic électrophorétique HbA_{1c} chevauche les pics d'HbA₁ₐ/HbA_{1b}.

### Exemple 3

Une électrophorèse capillaire a été réalisée à partir d'échantillons de référence (Exocell, USA), contenant différentes fractions d'hémoglobine glyquée purifiées (fractions A₀ et A_{1c} ou fractions A₀, A_{1b} et A₁ₐ), en utilisant une composition tampon contenant 200mM de CAPSO et 10mM de putrescine (pH10.20) mais pas de composé borate, ni de composé boronate. Les profils électrophorétiques standards A_{1a,b} et A_{1c} obtenus sont présentés en figure 3. Le pic correspondant à HbA_{1c} co-migre avec celui de HbAₒ.

### Exemple 4

Une électrophorèse capillaire a été réalisée à partir d'échantillons de référence (Exocell, USA), contenant différentes fractions d'hémoglobine glyquée purifiées (fractions A₀ et A_{1c} ou fractions A₀, A_{1b} et A₁ₐ), en utilisant une composition tampon contenant 200mM de CAPSO, 10mM de putrescine et 50mM de borate (pH 10.20). Les profils électrophorétiques standards A_{1a,b} et A_{1c} obtenus sont présentés en figure 4. Le pic correspondant à HbA_{1c} se trouve entre les pics correspondant à HbAₒ et HbA₁ₐ/HbA_{1b} et est trop proche du pic correspondant aux autres HbA₁ pour permettre un dosage fiable de HbA_{1c}.

### Exemple 5

Une électrophorèse capillaire a été réalisée à partir d'échantillons de référence (Exocell, USA), contenant différentes fractions d'hémoglobine glyquée purifiées (fractions A₀ et A_{1c} ou fractions A₀, A_{1b} et A₁ₐ), en utilisant une composition tampon contenant 200mM de CAPSO, 10mM de putrescine et 50mM d'acide 3-CarboxyPhenylBoronique (pH 10.20). Les profils électrophorétiques standards A_{1a,b} et A_{1c} obtenus sont présentés en figure 5. Le pic correspondant à HbA_{1c} se trouve entre les pics correspondant à HbA_{1b} et HbA₁ₐ.

### Exemple 6

Une électrophorèse capillaire a été réalisée à partir d'échantillons de référence (Exocell, USA), contenant différentes fractions d'hémoglobine glyquée purifiées (fractions A₀ et A_{1c} ou fractions A₀, A_{1b} et A₁ₐ), en utilisant une composition tampon contenant 200mM de CAPSO, 10mM de DAB et 50mM d'acide 3,5-diCarboxyPhenylBoronique (pH 10.20). Les profils électrophorétiques standards A_{1a,b} et A_{1c} obtenus sont présentés en figure 6. Le pic correspondant à HbA_{1c} se trouve après les pics correspondant à HbA_{1b} et HbA₁ₐ.

### Exemple 7

Une électrophorèse capillaire a été réalisée à partir d'échantillons de référence (Exocell, USA) contenant différentes fractions d'hémoglobine glyquée purifiées (fractions A₀ et A_{1c} ou fractions A₀, A_{1b} et A₁ₐ), en utilisant une composition tampon contenant 200mM de CAPSO, 15mM de ralentisseur de flux (putrescine) et 100mM d'acide 3,5-diCarboxyPhenylBoronique (pH 10.20). Les profils électrophorétiques standards A_{1a,b} et A_{1c} obtenus sont présentés en figure 7. On constate que le pic correspondant à HbA_{1c} se trouve après les pics correspondant à HbA_{1b} et HbA₁ₐ et est bien distinct de ces pics ; la séparation entre l'hémoglobine HbA_{1c} et les hémoglobines HbA₁ₐ et HbA_{1b} est excellente.

### Exemple 8

Une électrophorèse capillaire a été réalisée à partir d'échantillons de référence contenant de l'HbA₀ et/ou différentes fractions d'hémoglobine glyquée purifiées (fractions A₀ et A_{1c} ou fractions A₀, A_{1b} et A₁ₐ), en utilisant une composition tampon contenant 200mM de CHES, 20mM de ralentisseur de flux (putrescine) et 30mM d'acide 3,5-diCarboxyPhenylBoronique (à pH : 9.40). Les profils électrophorétiques standards A₀, A_{1a,b} et A_{1c} obtenus sont présentés en figure 8. On constate que le pic correspondant à HbA_{1c} se trouve après les pics correspondant à HbA₀, HbA_{1b} et HbA₁ₐ et est bien distinct de ces pics ; la séparation entre l'hémoglobine HbA_{1c} et les hémoglobines HbA₁ₐ et HbA_{1b} est excellente.

### Exemple 9

Une électrophorèse capillaire a été réalisée à partir d'échantillons de référence contenant de l'HbA₀ et/ou différentes fractions d'hémoglobine glyquée purifiées (fractions A₀ et A_{1c} ou fractions A₀, A_{1b} et A₁ₐ), en utilisant une composition tampon contenant 200mM de CHES, 20mM de ralentisseur de flux (putrescine) et 30mM d'acide 3,4-diCarboxyPhenylBoronique (à pH : 9.40). Les profils électrophorétiques standards A₀, A_{1a,b} et A_{1c} obtenus sont présentés en figure 9. On constate que le pic correspondant à HbA_{1c} se trouve après les pics correspondant à HbA₀, HbA_{1b} et HbA₁ₐ et est bien distinct de ces pics ; la séparation entre l'hémoglobine HbA_{1c} et les hémoglobines HbA₁ₐ et HbA_{1b} est excellente.

En comparant les profils électrophorétiques des exemples 8 et 9, on constate que l'acide 3,5-diCarboxyPhenylBoronique permet d'obtenir un résultat légèrement meilleur en termes de séparation de l'HbA_{1c} par rapport aux autres fractions, alors que l'acide 3,4-diCarboxyPhenylBoronique permet d'obtenir un résultat légèrement meilleur en termes de focalisation.

### Exemple 10

Une électrophorèse capillaire a été réalisée sur Capillarys® (Sebia) à partir d'échantillons de référence (Exocell, USA) contenant différentes fractions d'hémoglobine glyquée purifiées (fractions A₀ et A_{1c} ou fractions A₀, A_{1b} et A₁ₐ), en utilisant une composition tampon contenant 200mM de tampon CHES, 20mM de putrescine et 30mM d'acide 3,5-diCarboxyPhenylBoronique, à pH 9.40. Les profils électrophorétiques standards A_{1a,b} et A_{1c} obtenus sont présentés en figure 10. On constate que le pic correspondant à HbA_{1c} se trouve après les pics correspondant à HbA_{1b} et HbA₁ₐ et est bien distinct de ces pics ; la séparation entre l'hémoglobine HbA_{1c} et les hémoglobines HbA₁ₐ et HbA_{1b} est excellente.

### Exemple 11

Des analyses par électrophorèse capillaire ont été réalisées à partir d'un sang humain quelconque dilué au 1/6^{ième} dans la solution hémolysante (eau + 1g/L de Triton X100), en utilisant une composition tampon contenant soit 200mM de tampon CAPSO, 10mM de putrescine et 50mM d'acide 3,5-diCarboxyPhenylBoronique, à pH 10.20, soit 200mM de tampon CHES, 20mM de putrescine et 30mM d'acide 3,5-diCarboxyPhenylBoronique, à pH 9.40. Les électrophérogrammes obtenus sur Capillarys® (Sebia) en utilisant un capillaire en silice fondue, non coaté, sont présentés en figures 11A et 11B, respectivement. On observe dans les 2 cas une séparation parfaite de l'hémoglobine HbA_{1c} des autres formes d'hémoglobines.

### Exemple 12

On a étudié l'influence de la concentration en acide 3,5-diCarboxyPhenylBoronique dans la composition tampon sur la séparation entre les hémoglobines A_{1c} et A₀. La composition tampon utilisée contenait 200mM de CAPSO, 15mM de Putrescine et 0 à 120mM de 3,5-diCarboxyPhenylBoronique. Les résultats sont présentés en figure 12. On constate que la séparation A_{1c} / A₀ augmente avec la concentration en acide 3,5-diCarboxyPhenylBoronique.

### Exemple 13

Des électrophorèses capillaires ont été réalisées sur Capillarys® (Sebia) à partir de quatre échantillons différents dilués au 1/6^{ième} dans la solution hémolysante (eau + 1g/L de Triton X100): du sang humain quelconque comprenant un variant HbE (figure 13), un contrôle AFSC (figure14), un pool de sangs quelconques comportant des variants F et Bart's (figure 15) et un sang humain quelconque comprenant un variant HbD (figure 16). L'électrophorèse capillaire a été réalisée dans une composition tampon contenant 200mM de tampon CHES, 20mM de putrescine et 30mM d'acide 3,5-diCarboxyPhenylBoronique, à pH 9.40, en utilisant un capillaire en silice fondue, non coaté.

Les figures 13-16 montrent l'absence d'interférences des principaux variants de l'hémoglobine (E, F, S, C, D et Bart) avec la fraction HbA_{1c}. On note toutefois que dans le cas l'hémoglobine Bart, la résolution n'est pas totale entre les fractions Hb Bart et HbA_{1c}. Par conséquent, pour pouvoir doser l'HbA_{1c} en présence d'Hb Bart, il faut pouvoir quantifier ces deux fractions grâce à une méthode d'intégration adaptée. Si cela n'est pas possible, il sera nécessaire d'alerter l'utilisateur à ce sujet, dans l'éventualité où il observerait ce type de profil avec épaulement sur le pic attendu.

### Exemple 14

Les résultats obtenus par électrophorèse capillaire par la méthode de la présente invention en utilisant l'analyseur Capillarys® (Sebia) ont été comparés aux résultats obtenus avec une des techniques de référence : l'HPLC avec l'analyseur Variant II Turbo® (Bio-Rad).

L'électrophorèse capillaire a été réalisée à partir de sangs totaux dilués au 1/6^{ième} dans la solution hémolysante (eau + 1g/L de Triton X100), dans une composition tampon contenant 200mM de tampon CHES, 20mM de putrescine et 30mM d'acide 3,5-diCarboxyPhenylBoronique, à pH 9.40.

La figure 17 montre la très bonne corrélation de cette nouvelle technique d'analyse par électrophorèse capillaire avec l'analyse de l'HbA_{1c} par HPLC avec le Variant II turbo® de Bio-Rad. Après calibration des données EC en utilisant 2 calibrateurs (A1_{c} faible et A_{1c} forte), les valeurs obtenues par la méthode de la présente invention sont très proches de celles obtenues par la méthode de référence.

### Exemple 15 : Etude démontrant l'absence d'interférence de la fraction labile de l'HbA_{1c} avec le dosage de l'HbA_{1c} par la méthode de la présente invention.

Dans l'hypothèse où le composé complexant le glucose et apportant des charges négatives à pH alcalin mis en oeuvre dans le cadre de la méthode d'analyse de la présente invention serait capable d'interagir avec le glucose sanguin (cette interaction est hypothétique et non démontrée), une étude de l'éventuelle interférence du glucose libre sur le résultat du dosage de l'HbA_{1c} a été réalisée de la manière suivante : un sang quelconque a été incubé 3 heures à 37°C avec différentes concentrations de glucose (de 0 à 50g/L), de manière à créer de la forme labile de l'HbA_{1c} (forme obtenue avant réarrangement de la molécule (réarrangement d'Amadori)). Une fois l'incubation réalisée, les échantillons de sangs ont été centrifugés et les culots obtenus ont été reconstitués dans de l'eau physiologique et la solution hémolysante (15µL de culot + 25µL d'eau physiologique + 160µL de solution hémolysante (eau + 1g/L Triton X100)) puis analysés parallèlement sur automate Hydrasys® de Sebia (gel HbA_{1c}) et en technique Capillarys® (Sebia) en utilisant la composition tampon contenant 200mM de tampon CHES, 20mM de putrescine et 30mM d'acide 3,5-diCarboxyPhenylBoronique, à pH 9.40.

Le gel HbA_{1c} obtenu par l'analyse sur automate Hydrasys® (voir figure 18A) permet de confirmer la formation de fraction labile de l'HbA_{1c} migrant au même niveau que l'HbA_{1c} et en concentration croissante à mesure que la concentration en glucose augmente lors de l'incubation avec le sang. A noter qu'en gel, dans les conditions normales d'utilisation définies par Sebia, la fraction labile n'apparaîtrait pas, notamment en raison du pH acide de la solution hémolysante.

Les analyses effectuées sur Capillarys® (Sebia) avec une composition tampon de l'invention (200mM de tampon CHES, 20mM de putrescine et 30mM d'acide 3,5-diCarboxyPhenylBoronique, à pH 9.40), sur les mêmes échantillons de sangs, montrent à l'inverse que le dosage n'est pas perturbé par la présence de glucose libre, quelle que soit la concentration de glucose incubé, dans la gamme étudiée (0 à 50g/L) : les profils et les valeurs d'HbA1c sont inchangés (voir figures 18B et 18C).

### REFERENCES

Abraham, E.C. ; Cameron, B.F. ; Abraham, A. ; Stallings, M., "Glycosylated hemoglobins in heterozygotes ans homozygotes for hemoglobin C with or without diabetes", Journal of Laboratory and Clinical Medicine, 104, 602-609, 1984.
Beccaria, L.; Chiumello, G. ; Gianazza, E. ; Luppis, B.; Righetti, PG., "Hemoglobin A1c séparation by isoelectric focusing", American Journal of Hematology, 4, 367-374, 1978.
Bosisio, A.; Righetti, P., "Determination of glycosylated haemoglobin by isoelectric focusing in non-linear pH gradients", Journal of Chromatography, 307, 103-110, 1984.
Doelman, C; Siebelder, C; Nijhof, W.; Weykamp, W.; Janssens, J.; Penders, T., "Capillary electrophoresis system for hemoglobin A1c determinations evaluated", Clînical Chemistry, 43, 4, 644-648, 1997.
Hempe, JM.; Craver, RD., "Quantification of hemoglobin variants by capillary isoelectric focusing", Clinical Chemistry, 40,12, 2288-2295, 1994.
Hempe, JM. ; Granger, JN. ; Craver, RD., "Capillary isoelectric focusing of hemoglobin variants in the pediatrie clinical laboratory", Electrophoresis, 18, 1785-1795, 1997.
Janssens, J., *"Capillary electrophoresis detection and*/*or analysis method and unit",* EP 0 733 900 A2, 1996.
Janssens, J.; Chevigné, P.; Louis, P., *"Capillary electrophoresis method using initialized capillary and polyanion-containing buffer and chemical kit therefore",* US Patent 5,611,903, 1997.
Menard, L; Dempsey, M.; Blankstein, L; Aleyassine, H.; Wacks, M,; Soeldner, J., "Quantitative determination of glycosylated hemoglobin A1 by agar gel electrophoresis", Clinical Chemistry, 26, 11, 1598-1602,1980.
Molteni, S.; Frîschknecht, H.; Thormann, W., "Application of dynamic capillary isoelectric focusing to the analysis of human hemoglobin variants", Electrophoresis, 15, 22-30, 1994.
Panteghini, M. John, W.G., on behalf of the IFCC Scientific Division, « Implementation of haemoglobin A1c results traceable to the IFCC reference system: the way forward.", Clin Chem Lab Med., 45(8), 942-4, 2007.
Simon, M.; Cuan J., "Hemoglobin A1c by isoelectric focusing", Clinical Chemistry, 28,1, 9-12, 1982.
Siren, H.; Laitinen, P.; Turpeinen, U.; Karppinen, P., "Direct monitoring of glycohemoglobin A1c in the blood samples of diabetic patients by capillary electrophoresis. Comparison with an immunoassay method", Journal of Chromatography A, 979, 201-207, 2002.
Stickland, M.; Perkins, C; Wales, J" "The measurement of haemoglobin A1c by isolectric focusing in diabetic patients", Diabetologia, 22, 315-317,1982.
Wilson, D.H., Bogacz, J.P., Forsythe, C.M., Turk, P.J., Lane, T.L., Gates, R.C., Brandt, D.R. "Fully automated assay of glycohemoglobin with the Abbott IMx analyzer: novel approaches for separation and detection", Clin Chem., 39(10), 2090-7, 1993.

## Revendications

1. Méthode d'analyse par électrophorèse capillaire d'une ou plusieurs hémoglobine(s) glyquée(s) comportant au moins une chaîne de globine bêta comportant un résidu glucose lié à l'acide aminé se trouvant en position N-terminale, lesdites une ou plusieurs hémoglobine(s) glyquée(s) étant contenue(s) dans un échantillon biologique comprenant des hémoglobines, ladite méthode comprenant les étapes suivantes :
a. l'introduction, dans un capillaire d'électrophorèse, d'une composition tampon et de l'échantillon biologique ; la composition tampon comprenant au moins un composé qui complexe spécifiquement les résidus glucose liés à un acide aminé se trouvant en position N-terminale dans les hémoglobines glyquées de l'échantillon biologique et apporte à cette ou ces hémoglobine(s) glyquée(s) plusieurs charges électriques négatives à pH alcalin, ledit composé comprenant deux ou plus de deux groupements fonctionnels :
i. l'un au moins de ces groupements fonctionnels complexant spécifiquement un ou plusieurs résidu(s) glucose, apportant ainsi une charge électrique négative par résidu glucose complexé, et
ii. l'autre, un des autres ou les autres groupement(s) fonctionnel(s) ne complexant pas ledit ou lesdits résidu(s) glucose, conférant une ou plusieurs charge(s) électrique(s) négative(s) à pH alcalin à ladite ou auxdites hémoglobine(s) glyquée(s),
b. la séparation des constituants de l'échantillon biologique par électrophorèse capillaire; et
c. la détection d'une ou plusieurs hémoglobine(s) présente(s) dans l'échantillon biologique et la génération d'un électrophorégramme à partir du signal de détection, qui est proportionnel à la quantité d'hémoglobine(s) détectée.

2. Méthode selon la revendication 1, pour l'analyse par électrophorèse capillaire de l'hémoglobine A_{1c} contenue dans un échantillon biologique comprenant des hémoglobines.

3. Méthode selon l'une quelconque des revendications 1 à 2, dans laquelle le composé complexant spécifiquement des résidus glucose d'hémoglobines glyquées de l'échantillon biologique et apportant des charges négatives à pH alcalin comporte un (ou plusieurs) groupement(s) boronyle et/ou boronate, et en particulier est :
(i) un composé boronate, de formule générale RB(OH)₂ ou RB(OH)₃⁻, dans laquelle le groupement R comprend au moins un aryle et/ou un alkyle (linéaire, ramifié ou cyclique) et/ou un aralkyle et/ou d'autres groupements fonctionnels ou hétéroatomes, et/ou une combinaison de ceux-ci, et ledit groupement R apporte une ou plusieurs charge(s) électriques(s) négative(s) à pH alcalin pour chaque résidu de glucose complexé avec le groupement boronate ; ou
(ii) un sel de ce composé boronate.

4. Méthode selon l'une quelconque des revendications 1 à 3, dans laquelle la concentration, dans la composition tampon, du composé complexant spécifiquement des résidus glucose d'hémoglobine(s) glyquée(s) de l'échantillon biologique et apportant des charges négatives à pH alcalin, est en excès stoechiométrique par rapport à la quantité totale de protéines, par rapport à la quantité totale de toutes les hémoglobines présentes dans l'échantillon biologique ou par rapport à la quantité totale de toutes les hémoglobines comportant du glucose présentes dans l'échantillon biologique.

5. Méthode selon l'une quelconque des revendications 1 à 4, dans laquelle la composition tampon comprend en outre un ralentisseur de flux, en particulier un ralentisseur de flux qui est une diamine aliphatique, plus particulièrement une diamine aliphatique choisie parmi les 1,3-diaminopropane, 1,4-diaminobutane (putrescine), 1,5-diaminopentane (cadavérine), 1-6-diaminohexane, la spermine et la DETA, ou un dérivé de cette diamine aliphatique, par exemple une polyamine aliphatique, une polyamine cyclique ou un sel de diamine aliphatique, ou un de leurs mélanges, et plus particulièrement encore un ralentisseur de flux qui est la putrescine ou un dérivé.

6. Méthode selon l'une quelconque des revendications 1 à 5, dans laquelle la composition tampon comprend en outre:
- un composé tampon ayant un pKa compris entre 8,0 et 11,0 ; et/ou
- une base ; et/ou
- un sel, en particulier un sel de sodium, par exemple du chlorure de sodium ; et/ou
- une solution de dilution appropriée, par exemple de l'eau.

7. Méthode selon l'une quelconque des revendications 1 à 6, dans laquelle la composition tampon a un pH supérieur ou égal à 9, de préférence entre 9,0 et 11,0 et plus préférablement un pH compris entre 9,0 et 10,0, par exemple un pH de 9,4.

8. Méthode selon l'une quelconque des revendications 1 à 7, dans laquelle :
- le composé complexant spécifiquement des résidus glucose d'hémoglobines glyquées et apportant des charges négatives à pH alcalin comporte un ou plusieurs groupements anionisables à pH alcalin, en particulier un ou plusieurs carboxylate(s), carboxyle(s), sulfonate(s) et/ou sulfonyle(s), et/ou
- le composé boronate tel que défini à la revendication 3 est :
- un phénylboronate polysubstitué,
- en particulier un phénylboronate disubstitué, par exemple par des groupements carboxyle(s) et/ou sulfonyle(s),
- plus particulièrement un composé dans lequel le groupement R est un diacide, de préférence un diacide carboxylique
- plus particulièrement encore un acide diCarboxyPhenylBoronique, choisi de préférence parmi l'acide 3,4-diCarboxyPhenylBoronique et l'acide 3,5-diCarboxyPhenylBoronique, plus préférablement l'acide 3,5-diCarboxyPhenylBoronique, et/ou
- la concentration, dans la composition tampon, du composé capable de complexer spécifiquement des résidus glucose d'hémoglobine(s) glyquée(s) et d'apporter des charges négatives à pH alcalin est comprise entre 0,10 et 100 mM, de préférence entre 10 et 60 mM, et plus préférablement entre 20 et 50 mM, par exemple 30 mM, et/ou
- la concentration, dans la composition tampon, du ralentisseur de flux tel que défini à la revendication 5 est comprise entre 0,10 et 40 mM, de préférence entre 10 et 30 mM et plus préférablement entre 15 et 25 mM, par exemple 20 mM, et/ou
- le composé tampon tel que défini à la revendication 6 est un composé zwitterionique, en particulier un composé choisi parmi l'AMP, l'AMPD, l'AMPSO, la bicine, le CABS, le CAPS, le CAPSO, le CHES, l'HEPBS, la méthylamine, le TABS, le TAPS, la taurine, la tricine et le Tris, par exemple le CHES, le CAPS ou le CAPSO, et/ou
- la concentration, dans la composition tampon, du composé tampon tel que défini à la revendication 6 est comprise entre 20 et 500 mM, plus préférablement entre 50 et 400 mM, et plus préférablement encore entre 150 et 300 mM, par exemple 200 mM.

9. Méthode selon l'une quelconque des revendications 1 à 8, comprenant en outre :
- une étape de détermination, en particulier à partir d'un électrophorégramme tel que défini à la revendication 1, de la quantité d'une ou plusieurs hémoglobine(s) présente(s) dans l'échantillon biologique et/ou de la proportion d'une ou plusieurs hémoglobine(s) présente(s) dans l'échantillon biologique par rapport à la quantité totale de protéines, à la quantité totale d'hémoglobine ou à la quantité de certaines hémoglobines présentes dans l'échantillon biologique, et/ou
- une étape de quantification d'une ou plusieurs hémoglobine(s) présente(s) dans l'échantillon biologique par rapport à un ou plusieurs calibrateur(s) standardisé(s).

10. Méthode selon l'une quelconque des revendications 1 à 9, dans laquelle l'échantillon biologique est un échantillon de sang, en particulier un échantillon de sang normal ou non, lavé, décanté, centrifugé ou total, ledit échantillon étant, le cas échéant, hémolysé.

11. Méthode selon l'une quelconque des revendications 1 à 10, dans laquelle l'échantillon biologique est dilué dans une solution hémolysante, en particulier une solution hémolysante choisie parmi le groupe constitué de :
- la solution hémolysante Capillarys® Hemoglobin(e) ;
- la solution hémolysante Hydragel® HbA_{1c} ;
- la solution hémolysante MlNlCAP® Hemoglobin(e) ;
- l'eau pure ;
- l'eau additionnée de tensioactif, en particulier l'eau additionnée de triton ; et
- leurs mélanges.

12. Composition tampon appropriée pour l'analyse, par électrophorèse capillaire, d'une ou plusieurs hémoglobines glyquées comportant un résidu glucose lié à l'acide aminé se trouvant en position N-terminale sur les chaînes de globine bêta, contenues dans un échantillon biologique comprenant une ou plusieurs hémoglobine(s), ladite composition comprenant au moins un composé qui complexe spécifiquement les résidus glucose liés à un acide aminé se trouvant en position N-terminale d'hémoglobines glyquées, et apportant à ces hémoglobines glyquées plusieurs charges électriques négatives à pH alcalin, ladite composition étant **caractérisée en ce que** le composé, qui comporte un (ou plusieurs) groupement(s) boronyle et/ou boronate, est :
(i) un composé boronate, de formule générale RB(OH)₂ ou RB(OH)₃⁻, dans laquelle le groupement R comprend au moins un aryle et/ou un alkyle (linéaire, ramifié ou cyclique) et/ou un aralkyle et/ou d'autres groupements fonctionnels ou hétéroatomes, et/ou une combinaison de ceux-ci, et ledit groupement R apporte deux ou plus de deux charges électriques négatives à pH alcalin pour chaque résidu de glucose complexé avec le groupement boronate, en particulier le composé boronate est un phénylboronate polysubstitué ; ou
(ii) un sel de ce composé boronate.

13. Composition tampon selon la revendication 12, qui est telle qu'en outre définie dans l'une quelconque des revendications 4 à 8.

14. Trousse comprenant une composition tampon selon la revendication 12 ou 13 et, le cas échéant, une notice d'utilisation.

15. Utilisation d'une composition tampon telle que définie dans l'une quelconque des revendications 1 à 8, ou d'une trousse selon la revendication 14, pour la séparation, par électrophorèse capillaire, d'une ou plusieurs hémoglobine(s) glyquée(s) comportant au moins une chaîne de globine bêta comportant un résidu glucose lié à l'acide aminé se trouvant en position N-terminale, d'autres protéines, en particulier d'au moins une autre hémoglobine et de préférence des autres hémoglobines présente(s) dans un échantillon biologique.

16. Utilisation selon la revendication 15, pour le diagnostic du diabète chez un humain ou un mammifère non humain et/ou pour le suivi de l'équilibre glycémique d'un mammifère humain ou non humain.

## Patentansprüche

1. Verfahren zur Analyse eines oder mehrerer glykierter Hämoglobine mit mindestens einer Beta-Globinkette mit einem an die in N-Position befindliche Aminosäure gebundenen Glucoserest durch Kapillarelektrophorese, wobei ein oder mehrere glykierte Hämoglobine in einer biologischen Probe mit Hämoglobinen enthalten sind, wobei das Verfahren die folgenden Schritte umfasst:
a. die Einführung einer Pufferzusammensetzung und der biologischen Probe in eine Elektrophoresekapillare, wobei die Pufferzusammensetzung mindestens eine Verbindung umfasst, die speziell die Glucosereste komplexiert, die an eine Aminosäure gebunden sind, die sich in der N-terminalen Position in den glykierten Hämoglobinen der biologischen Probe befindet, und dem oder den glykierten Hämoglobinen mehrere negative elektrische Ladungen mit alkalischem pH-Wert zuführt, wobei die Verbindung zwei oder mehr funktionelle Gruppen umfasst:
i. wobei mindestens eine dieser funktionellen Gruppen einen oder mehrere Glucosereste spezifisch komplexiert, wodurch eine negative elektrische Ladung pro komplexiertem Glucoserest zugeführt wird, und
ii. die andere, eine der anderen oder die anderen funktionellen Gruppen, die den oder die Glucosereste nicht spezifisch komplexieren, der oder den glykierten Hämoglobinen eine oder mehrere negative elektrische Ladungen pro komplexiertem Glucoserest verleihen.
b. die Abscheidung der Bestandteile der biologischen Probe durch Kapillarelektrophorese und
c. den Nachweis eines oder mehrerer in der biologischen Probe enthaltenen Hämoglobine und die Erstellung eines Elektropherogramms aus dem Nachweissignal, das sich proportional zur Menge des/der nachgewiesenen Hämoglobins(e) verhält.

2. Verfahren nach Anspruch 1 zur Analyse des in einer biologischen Probe mit Hämoglobinen enthaltenen Hämoglobins A1c durch Kapillarelektrophorese.

3. Verfahren nach einem der Ansprüche 1 bis 2, in dem die Verbindung, die die Glucosereste glykierter Hämoglobine der biologischen Probe spezifisch komplexiert und negative Ladungen mit alkalischem pH-Wert liefert, eine (oder mehrere) Boronyl- und/oder Boronatgruppe(n) umfasst, und insbesondere:
(i) eine Boronatverbindung mit der allgemeinen Formel RB(OH)2 oder RB(OH)3" ist, in der die Gruppe R mindestens ein Aryl und/oder ein Alkyl (linear, verzweigt oder zyklisch) und/oder ein Aralkyl und/oder andere funktionelle Gruppen oder Heteroatome und/oder eine Kombination davon umfasst, und die Gruppe R jedem mit der Boronatgruppe komplexierten Glucoserest eine oder mehrere negative elektrische Ladung(en) mit alkalischem pH-Wert zuführt; oder
ii) ein Salz dieser Boronatverbindung ist.

4. Verfahren nach einem der Ansprüche 1 bis 3, in dem die Konzentration in der Pufferzusammensetzung der Verbindung, die spezifisch Glucosereste glykierter Hämoglobine der biologischen Probe komplexiert und negative Ladungen mit alkalischem pH-Wert zuführt, in stöchiometrischem Überschuss relativ zur Gesamtmenge an Proteinen, relativ zur Gesamtmenge aller in der biologischen Probe enthaltenen Hämoglobine oder relativ zur Gesamtmenge aller in der biologischen Probe enthaltenen glucosehaltigen Hämoglobine vorhanden ist.

5. Verfahren nach einem der Ansprüche 1 bis 4, in dem die Pufferzusammensetzung des Weiteren einen Strömungsverlangsamer umfasst, insbesondere einen Strömungsverlangsamer, der ein aliphatisches Diamin ist, insbesondere ein aliphatisches Diamin, das aus 1,3-Diaminopropan, 1,4-Diaminobutan (Putrescin), 1,5-Diaminopentan (Kadaverin), 1-6-Diaminohexan, Spermin und DETA oder einem Derivat dieses aliphatischen Diamins, beispielsweise ein aliphatisches Polyamin, cyclisches Polyamin oder aliphatisches Diaminsalz oder eine Mischung davon, ausgewählt wird, und insbesondere einen Strömungsverlangsamer, der Putrescin oder ein Derivat davon ist.

6. Verfahren nach einem der Ansprüche 1 bis 5, in dem die Pufferzusammensetzung des Weiteren folgendes umfasst:
- eine Pufferverbindung mit einem pKa zwischen 8,0 und 11,0; und/oder
- eine Base; und/oder
- ein Salz, insbesondere ein Natriumsalz, z.B. Natriumchlorid; und/oder
- eine geeignete Verdünnungslösung, z.B. Wasser.

7. Verfahren nach einem der Ansprüche 1 bis 6, in dem die Pufferzusammensetzung einen pH-Wert größer/gleich 9, vorzugsweise zwischen 9,0 und 11,0 und insbesondere einen pH-Wert zwischen 9,0 und 10,0, zum Beispiel einen pH-Wert von 9,4, hat.

8. Verfahren nach einem der Ansprüche 1 bis 7, in dem:
- die Verbindung, die spezifisch Glucosereste glykosehaltiger Hämoglobine komplexiert und negative Ladungen mit alkalischem pH-Wert zuführt, eine oder mehrere anionisierbare Gruppen mit alkalischem pH-Wert umfasst, insbesondere eines oder mehrere Carboxylat(e), Carboxyl(e), Sulfonat(e) und/oder Sulfonyl(e), und/oder
- die Boronatverbindung nach Anspruch 3:
∘ ein polysubstituiertes Phenylboronat ist,
∘ insbesondere ein Phenylboronat ist, das z.B. mit Carboxyl- und/oder Sulfonylgruppen disubstituiert ist,
∘ insbesondere eine Verbindung ist, in der die Gruppe R eine Disäure, vorzugsweise eine Dicarbonsäure, ist.
∘ und vorzugsweise insbesondere eine Dicarboxyphenylboronsäure ist, die vorzugsweise aus 3,4-Dicarboxyphenylboronsäure und 3,5-Dicarboxyphenylboronsäure ausgewählt wird und vorzugsweise 3,5-Dicarboxyphenylboronsäure ist, und/oder
- in der Pufferzusammensetzung die Konzentration der Verbindung, die in der Lage ist, Glucosereste von glykiertem Hämoglobin bzw. glykierten Hämoglobinen spezifisch zu komplexieren und negative Ladungen mit alkalischem pH-Wert zuzuführen, zwischen 0,10 und 100 mM, vorzugsweise zwischen 10 und 60 mM, und insbesondere zwischen 20 und 50 mM, z. B. bei 30 mM, liegt, und/oder
- in der Pufferzusammensetzung die Konzentration des Strömungsverlangsamers nach Anspruch 5 zwischen 0,10 und 40 mM, vorzugsweise zwischen 10 und 30 mM und insbesondere zwischen 15 und 25 mM, z.B. bei 20 mM, liegt und/oder
- die Pufferverbindung nach Anspruch 6 eine zwitterionische Verbindung ist, insbesondere eine Verbindung, die ausgewählt wird aus AMP, AMPD, AMPSO, Bicin, CABS, CAPS, CAPSO, CHES, HEPBS, Methylamin, TABS, TAPS, Taurin, Tricin und Tris, z.B. CHES, CAPS oder CAPSO, und/oder
- in der Pufferzusammensetzung die Konzentration der Pufferverbindung nach Anspruch 6 zwischen 20 und 500 mM, vorzugsweise zwischen 50 und 400 mM, und insbesondere zwischen 150 und 300 mM, z.B. bei 200 mM, liegt.

9. Verfahren nach einem der Ansprüche 1 bis 8, das des Weiteren folgendes umfasst:
- einen Schritt zur Bestimmung, insbesondere mithilfe eines Elektropherogramms nach Anspruch 1, der Menge eines oder mehrerer in der biologischen Probe enthaltenen Hämoglobine und/oder des Anteils eines oder mehrerer in der biologischen Probe enthaltenen Hämoglobine im Verhältnis zur Gesamtmenge an Proteinen, der Gesamtmenge an Hämoglobin oder der Menge bestimmter, in der biologischen Probe enthaltener Hämoglobine, und/oder
- ein Schritt der Quantifizierung eines oder mehrerer in der biologischen Probe enthaltenen Hämoglobine im Verhältnis zu einem oder mehreren standardisierten Kalibratoren.

10. Verfahren nach einem der Ansprüche 1 bis 9, **dadurch gekennzeichnet, dass** die biologische Probe eine Blutprobe, insbesondere eine Probe von normalem oder nicht-normalem, gewaschenem, geklärtem, zentrifugiertem Blut oder Vollblut ist, wobei diese Probe gegebenenfalls hämolysiert ist.

11. Verfahren nach einem der Ansprüche 1 bis 10, in dem die biologische Probe in einer hämolysierenden Lösung aufgelöst wird, insbesondere einer hämolysierenden Lösung, die ausgewählt wird aus der Gruppe bestehend aus:
- der hämolysierenden Lösung Capillarys® Hämoglobin;
- der hämolysierenden Lösung Hydragel® HbA1c;
- der hämolysierenden Lösung MINICAP® Hämoglobin(e) ;
- reinem Wasser;
- mit Tensiden versetztem Wasser, insbesondere mit Triton versetztem Wasser; und
- ihren Mischungen.

12. Pufferzusammensetzung, die sich zur Analyse durch Kapillarelektrophorese eines oder mehrerer glykierter Hämoglobine eignet, die einen Glucoserest umfassen, der an die in den Beta-Glykoseketten in N-Position befindliche Aminosäure gebunden ist, wobei diese in einer biologischen Probe mit einem oder mehreren Hämoglobinen enthalten sind, wobei die Zusammensetzung mindestens eine Verbindung umfasst, die die Glukosereste spezifisch komplexiert, die an eine Aminosäure gebunden sind, die sich in terminaler N-Position glykierter Hämoglobine befindet, und den glykierten Hämoglobinen mehrere negative elektrische Ladungen mit alkalischem pH-Wert zuführt, wobei die Zusammensetzung **dadurch gekennzeichnet ist, dass** die Verbindung, die eine (oder mehrere) Boronyl- und/oder Boronatgruppe(n) umfasst,:
(i) eine Boronatverbindung der allgemeinen Formel RB(OH)2 oder RB(OH)3" ist, in der die Gruppe R mindestens ein Aryl und/oder Alkyl (linear, verzweigt oder cyclisch) und/oder Aralkyl und/oder andere funktionelle Gruppen oder Heteroatome, und/oder eine Kombination davon umfasst, und die Gruppe R für jeden mit der Boronatgruppe komplexierten Glucoserest zwei oder mehrere negative elektrische Ladung(en) mit alkalischem pH-Wert liefert, wobei insbesondere die Boronatverbindung ein polysubstituiertes Phenylboronat ist; oder
ii) ein Salz dieser Boronatverbindung ist.

13. Pufferzusammensetzung nach Anspruch 12, die darüber hinaus der Definition in einem der Ansprüche 4 bis 8 entspricht.

14. Set, das eine Pufferzusammensetzung nach Anspruch 12 oder 13 und gegebenenfalls eine Gebrauchsanweisung umfasst.

15. Verwendung einer Pufferzusammensetzung nach einem der Ansprüche 1 bis 8 oder eines Sets nach Anspruch 14 zur Abscheidung eines oder mehrerer in einer biologischen Probe enthaltenen glykierten Hämoglobine, die mindestens eine Beta-Globinkette mit einem an die Aminosäure in N-terminaler Position gebundenen Glucoserest umfassen, anderer Proteine, insbesondere mindestens eines anderen Hämoglobins und vorzugsweise anderer Hämoglobine, durch Kapillarelektrophorese.

16. Verwendung nach Anspruch 15 zur Diagnose von Diabetes bei einem Menschen oder nichtmenschlichen Säugetier und/oder zur Überwachung der Blutzuckereinstellung eines menschlichen oder nichtmenschlichen Säugetiers.

## Claims

1. Method of analysing by capillary electrophoresis one or more glycated hemoglobin(s) having at least one beta globin chain having a glucose residue bound to the N-terminal amino acid, said one or more glycated hemoglobin(s) being contained in a biological sample comprising hemoglobins, said method comprising the following steps:
a.introducing into an electrophoresis capillary a buffer composition and the biological sample; the buffer composition comprising at least one compound which specifically complexes the glucose residues bound to an amino acid located in the N-terminal position in the glycated haemoglobin(s) of the biological sample and provides this/these glycated haemoglobin(s) with a plurality of negative electric charges at alkaline pH, said compound comprising two or more functional groups:
i. at least one of these functional groups specifically complexing one or more glucose residue(s), thereby providing a negative electrical charge per complexed glucose residue, and
ii. the other, one of the other or other functional group(s) not complexing said glucose residue(s), conferring one or more negative electrical charge(s) at alkaline pH on said glycated haemoglobin(s),
b. separating the components of the biological sample by capillary electrophoresis; and
c. detecting one or more haemoglobin(s) present in the biological sample and generating an electropherogram from the detection signal, which is proportional to the amount of haemoglobin(s) detected.

2. Method according to claim 1, for the analysis by capillary electrophoresis of hemoglobin A_{1c} contained in a biological sample comprising hemoglobins.

3. Method according to any of claims 1 to 2, wherein the compound specifically complexing glucose residues of glycated haemoglobins in the biological sample and providing negative charges at alkaline pH comprises one (or more) boronyl and/or boronate group(s), and in particular is:
(i) a boronate compound of the general formula RB(OH)₂ or RB(OH)₃⁻, wherein the R group comprises at least one aryl and/or alkyl (linear, branched or cyclic) and/or aralkyl and/or other functional groups or heteroatoms, and/or a combination thereof, and said R group provides one or more negative electrical charge(s) at alkaline pH for each glucose residue complexed with the boronate group; or
(ii) a salt of this boronate compound.

4. Method according to any one of claims 1 to 3, wherein the concentration in the buffer composition of the compound complexing specifically glucose residues of glycated hemoglobin(s) in the biological sample and providing negative charges at alkaline pH is in stoichiometric excess relative to the total amount of protein, relative to the total amount of all hemoglobins present in the biological sample or relative to the total amount of all glucose-containing hemoglobins present in the biological sample.

5. Method according to any of claims 1 to 4, wherein the buffer composition further comprises a flow retarder, in particular a flow retarder which is an aliphatic diamine, more particularly an aliphatic diamine selected from 1,3-diaminopropane, 1,4-diaminobutane (putrescine), 1,5-diaminopentane (cadaverine), 1 -6-diaminohexane, spermine and DETA, or a derivative of this aliphatic diamine, for example an aliphatic polyamine, cyclic polyamine or aliphatic diamine salt, or a mixture thereof, and more particularly a flow retarder which is putrescine or a derivative thereof.

6. Method according to any of claims 1 to 5, wherein the buffer composition further comprises:
- a buffer compound having a pKa between 8.0 and 11.0; and/or
- a base; and/or
- a salt, in particular a sodium salt, e.g. sodium chloride; and/or
- a suitable dilution solution, e.g. water.

7. Method according to any of claims 1 to 6, wherein the buffer composition has a pH of 9 or more, preferably between 9.0 and 11.0 and more preferably a pH between 9.0 and 10.0, for example a pH of 9.4.

8. Method according to one of claims 1 to 7, wherein:
- the compound specifically complexing glucose residues of glycated haemoglobins and providing negative charges at alkaline pH comprises one or more groups anionizable at alkaline pH, in particular one or more carboxylate(s), carboxyl(s), sulphonate(s) and/or sulphonyl(s), and/or
- the boronate compound as defined in claim 3 is:
- a polysubstituted phenylboronate,
- in particular a phenylboronate disubstituted, for example with carboxyl(s) and/or sulphonyl(s) groups,
- more particularly a compound in which the group R is a diacid, preferably a dicarboxylic acid
- more particularly still a diCarboxyPhenylBoronic acid, preferably selected from 3,4-diCarboxyPhenylBoronic acid and 3,5-diCarboxyPhenylBoronic acid, more preferably 3,5-diCarboxyPhenylBoronic acid, and/or
- the concentration, in the buffer composition, of the compound capable of specifically complexing glucose residues of glycated haemoglobin(s) and of providing negative charges at alkaline pH is between 0.10 and 100 mM, preferably between 10 and 60 mM, and more preferably between 20 and 50 mM, e.g. 30 mM, and/or
- the concentration in the buffer composition, of the flow retarder as defined in claim 5 is between 0.10 and 40 mM, preferably between 10 and 30 mM and more preferably between 15 and 25 mM, e.g. 20 mM, and/or
- the buffer compound as defined in claim 6 is a zwitterionic compound, in particular a compound selected from AMP, AMPD, AMPSO, bicine, CABS, CAPS, CAPSO, CHES, HEPBS, methylamine, TABS, TAPS, taurine, tricine and Tris, e.g. CHES, CAPS or CAPSO, and/or
- the concentration, in the buffer composition, of the buffer compound as defined in claim 6 is between 20 and 500 mM, more preferably between 50 and 400 mM and even more preferably between 150 and 300 mM, e.g. 200 mM.

9. Method according to one of claims 1 to 8, further comprising:
- a step of determining, in particular from an electropherogram as defined in claim 1, the amount of one or more haemoglobin(s) present in the biological sample and/or the proportion of one or more haemoglobin(s) present in the biological sample relative to the total amount of protein, the total amount of haemoglobin or the amount of certain haemoglobins present in the biological sample, and/or
- a step of quantification of one or more haemoglobin(s) present in the biological sample relative to one or more standardised calibrator(s).

10. Method according to any one of claims 1 to 9, wherein the biological sample is a blood sample, in particular a normal or non-normal, washed, decanted, centrifuged or total blood sample, said sample being, where appropriate, hemolyzed.

11. Method according to any one of claims 1 to 10, wherein the biological sample is diluted in a hemolyzing solution, in particular a hemolyzing solution selected from the group consisting of:
- Capillarys® Hemoglobin hemolyzing solution;
- Hydragel® HbA_{1c} hemolyzing solution;
- MINICAP® Hemoglobin hemolyzing solution;
- pure water;
- surfactant-added water, in particular water with triton; and
- their mixtures.

12. Buffer composition suitable for the analysis, by capillary electrophoresis, of one or more glycated haemoglobins having a glucose residue bound to the amino acid located at the N-terminal position on the beta globin chains, contained in a biological sample comprising one or more haemoglobin(s), said compound comprising at least one compound which specifically complexes the N-terminal amino acid-bound glucose residues of glycated haemoglobins and provides these glycated haemoglobins with a plurality of negative electric charges at alkaline pH, said composition being **characterized in that** the compound, which comprises one (or more) boronyl and/or boronate group(s), is:
(i) a boronate compound of the general formula RB(OH)₂ or RB(OH)₃⁻, wherein the R group comprises at least one aryl and/or alkyl (linear, branched or cyclic) and/or aralkyl and/or other functional groups or heteroatoms, and/or a combination thereof, and said R group provides two or more negative electrical charges at alkaline pH for each glucose residue complexed with the boronate group, in particular the boronate compound is a polysubstituted phenylboronate; or
(ii) a salt of this boronate compound.

13. Buffer composition according to claim 12, which is as further defined in any of claims 4 to 8.

14. Kit comprising a buffer composition as claimed in claim 12 or 13 and, where appropriate, an instruction leaflet.

15. Use of a buffer composition as defined in any one of claims 1 to 8, or a kit according to claim 14, for the separation, by capillary electrophoresis, of one or more glycated haemoglobin(s) comprising at least one beta globin chain having an amino acid-bound glucose residue in the N-terminal position, from other proteins, in particular from at least one other haemoglobin and preferably other haemoglobins present in a biological sample.

16. Use according to claim 15, for the diagnosis of diabetes in a human or non-human mammal and/or for monitoring the glycemic control of a human or non-human mammal.
